(19) Europäisches Patentamt / European Patent Office / Office européen des brevets

(11) EP 1 427 818 B1

(12) **FASCICULE DE BREVET EUROPEEN**

(45) Date de publication et mention de la délivrance du brevet:
**20.02.2019 Bulletin 2019/08**

(21) Numéro de dépôt: **02722349.4**

(22) Date de dépôt: **18.03.2002**

(51) Int Cl.:
***C12N 9/10*** *(2006.01)*

(86) Numéro de dépôt international:
**PCT/FR2002/000951**

(87) Numéro de publication internationale:
**WO 2002/074943 (26.09.2002 Gazette 2002/39)**

(54) **MOLECULES D'ACIDES NUCEIQUES CODANT UNE DEXTRANE-SACCHARASE CATALYSANT LA SYNTHESE DE DEXTRANE PORTANT DES RAMIFICATIONS DE TYPE ALPHA-1,2 OSIDIQUES**

NUCLEINSÄUREMOLEKÜLE CODIEREND FÜR EINE DEXTRANSACCHARASE DIE DIE SYNTHESE VON DEXTRANE MIT TYP ALPHA-1,2 OSIDIC VERZWEIGUNGEN KATALYSIEREN

NUCLEIC ACID MOLECULES CODING FOR A DEXTRAN-SACCHARASE CATALYSING THE SYNTHESIS OF DEXTRAN WITH ALPHA 1,2-OSIDIC SIDECHAINS

(84) Etats contractants désignés:
**AT BE CH CY DE DK ES FI FR GB GR IE IT LI LU MC NL PT SE TR**

(30) Priorité: **16.03.2001 FR 0103631**
**19.12.2001 FR 0116495**

(43) Date de publication de la demande:
**16.06.2004 Bulletin 2004/25**

(60) Demande divisionnaire:
**12152893.9 / 2 481 803**

(73) Titulaire: **Institut National des Sciences Appliquées (INSA)**
**31077 Toulouse Cedex 4 (FR)**

(72) Inventeurs:
- **Pierre Monsan**
**31700 Mondonville (FR)**
- **Magali Remaud**
**31520 Ramonville (FR)**
- **Sophie Bozonnet**
**31150 Gagnac sur Garonne (FR)**
- **René-Mrac Willemot**
**31450 Pompertuzat (FR)**

(74) Mandataire: **Desaix, Anne et al**
**Ernest Gutmann - Yves Plasseraud S.A.S.**
**3, rue Auber**
**75009 Paris (FR)**

(56) Documents cités:
**EP-A- 0 325 872** **WO-A-00/47727**
**WO-A-89/12386**

- **DATABASE SWALL [Online] 1 May 1999 (1999-05-01) BHATNAGAR ET ALO.: "Dextransucrase" retrieved from EBI, accession no. Q9ZAR4 Database accession no. Q9ZAR4 XP002184801 -& DATABASE EM_PRO [Online] EMBL; 2 February 1999 (1999-02-02) BHATNAGAR ET AL.: "Leuconostoc mesenteroides dextransucrase (DEX) gene, complete cds" retrieved from EBI, accession no. U81374 Database accession no. U81374 XP002187315**
- **MONCHOIS V ET AL: "Cloning and sequencing of a gene coding for a novel dextransucrase from Leuconostoc mesenteroides NRRL B-1299 synthesizing only alpha(1-6) and alpha(1-3) linkages" GENE, vol. 182, no. 1-2, 5 December 1996 (1996-12-05), pages 23-32, XP004071926 BARKING, GB ISSN: 0378-1119**
- **KIM D ET AL: "DEXTRANSUCRASE CONSTITUTIVE MUTANTS OF LEUCONOSTOC MESENTEROIDES B-1299" ENZYME AND MICROBIAL TECHNOLOGY, STONEHAM, MA, US, vol. 17, no. 12, 1995, pages 1050-1056, XP001051263 ISSN: 0141-0229**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Remarques:

Le dossier contient des informations techniques présentées postérieurement au dépôt de la demande et ne figurant pas dans le présent fascicule.

## Description

La présente demande relève du domaine de la glycotechnologie et plus particulièrement de la synthèse d'oligosaccharides ou oligosides à effet prébiotique, thérapeutique ou diagnostique.

La présente demande divulgue des molécules d'acides nucléiques codant une enzyme ayant une activité de glycosyltransférase catalysant la synthèse de dextranes ou d'oligosides portant des ramifications de type $\alpha(1 \rightarrow 2)$ osidiques.

La demande divulgue également les enzymes synthétisées par les acides nucléiques ici décrits, ainsi que sur leurs systèmes d'expression dans des cellules procaryotes ou eucaryotes. Elle divulgue enfin l'utilisation desdites enzymes dans la production d'oligosaccharides dans l'alimentation, ou en tant que principe actif de produits thérapeutiques et/ou cosmétiques. L'invention porte plus particulièrement sur des polypeptides isolés tels que définis en revendications 1 et 2, sur des acides nucléiques isolés tels que définis en revendications 3 et 4, des vecteurs tels que définis en revendications 5 et 6, des cellules selon les revendications 7 et 8, des procédés de production d'une glycosyltransférase selon les revendications 9 à 11 et l'utilisation de la glycosyltransférase obtenue dans la fabrication d'une composition pharmaceutique ou cosmétique, selon la revendication 12.

Les oligosides et hétérooligosides jouent le rôle de signaux de reconnaissance et d'effecteur chez l'animal comme dans les plantes (on parle alors d'oligosaccharines), en se liant spécifiquement à des lectines, des glycosyltransférases, des glycosidases, des molécules d'adhésion, etc... Ainsi, les déterminants antigéniques des groupes sanguins sont des osides, et notre défense contre nombre de bactéries pathogènes est dirigée contre les structures osidiques de l'enveloppe bactérienne. Par ailleurs, l'une des raisons majeures du rejet des xénogreffes est l'existence de structures osidiques propres à chaque espèce. Ces propriétés, ainsi que les connaissances acquises ces dernières années sur les glycosyltransférases et les lectines, contribuent à faire de certains oligosides des candidats de choix pour la thérapeutique ou la prophylaxie des désordres liés à l'équilibre microbiologique de différents organes tels l'intestin, ou la peau. Par exemple, les oligosides constituent une alternative intéressante à l'utilisation de microorganismes et d'antibiotiques pour réguler la composition de la flore intestinale (effet prébiotique). Certains oligosides peuvent être considérés comme des "fibres solubles" lorsqu'ils ne sont pas métabolisés par les enzymes digestives humaines et animales ; en gagnant le côlon, ils interagissent avec la flore microbienne et affectent spécifiquement la croissance et l'adhésion de certaines espèces. Incorporées à faible dose (moins de 1 %) dans l'alimentation, certaines de ces molécules osidiques améliorent l'état de santé et stimulent la prise de poids des animaux.

Une revue des différentes glycosyltransférases, leur structure et leur activité, est décrite dans Vincent Monchois *et al.* (1). Brièvement :

a) Il apparaît que la structure des glycosyltransférases et/ou dextrane-saccharases étudiées est très conservée et est constituée, partant de la partie aminée de la protéine, d'une séquence signal, d'un domaine variable, d'un domaine catalytique et d'un domaine de liaison au glucane.

b) Les glucooligosides (GOS) sont synthétisables par des glycosyltransférases telles les dextrane-saccharases, à partir de substrats peu coûteux tel le saccharose et en présence d'un sucre accepteur de glucose. D'autres substrats, tels l'a-D-fluoro-glucose, le paranitrophényl-α-D-glucopyranoside, l'α-D-glucopyranoside-α-D-sorbofuranoside ou le 4-O-α-D-galactopyranosylsucrose peuvent également être utilisés.

Ces enzymes catalysent à partir du substrat le transfert d'unités glucose sur des molécules acceptrices. En présence d'un accepteur de glucose tel le maltose, ou l'isomaltose, les glycosyltransférases catalysent la synthèse d'oligosaccharides de bas poids moléculaire comprenant majoritairement des chaînes de 3 à 7 glucoses, selon la réaction :

$$\text{saccharose + accepteur} \rightarrow \text{accepteur glucosylé + fructose}$$

En pareil cas, les enzymes présentent généralement une spécificité de synthèse de liaisons osidiques conformes à celle formant le polymère donneur.

En revanche, en absence d'accepteur, l'enzyme synthétise des glucanes de haut poids moléculaire de type dextrane par transferts successifs d'unités α-D-glucopyranosyles à partir de saccharose, conformément à la réaction :

$$\text{n saccharose} \rightarrow (\text{glucose})_n + \text{n fructose}$$

c) Les structures et la fonction des glucanes ou des oligosides synthétisés par les glycosyltransférases dépendent de la souche bactérienne productrice.

Dans l'ensemble de ce texte, on appellera de façon générique des glycosyltransférases les différentes enzymes capables de catalyser la synthèse de polymères de glucose à partir de saccharose. Elles sont généralement produites par des souches bactériennes de type *Leuconostoc*, *Lactococcus*, *Streptococcus* ou *Neisseria*. La taille et la structure des glucanes produits dépendent de la souche productrice.

Les unités de glucose sont couplées par des liaisons osidiques $\alpha(1\rightarrow6)$ comme dans le dextrane, par des liaisons $\alpha(1\rightarrow3)$, comme dans le cas du mutane, ou par une alternance des deux types (alternane).

De la même façon, l'existence et la nature des ramifications, leur longueur et leur position varient selon l'origine de la souche productrice.

Les glycosyltransférases produisant des glucanes ou des GOS contenant au moins 50 % de liaison $\alpha(1\rightarrow6)$ sont appelées dextrane-saccharases. Les GOS synthétisés par ces enzymes portent, le cas échéant, des ramifications $\alpha(1\rightarrow2)$, $\alpha(1\rightarrow3)$ et/ou $\alpha(1\rightarrow4)$. Lesdites dextrane-saccharases sont produites notamment par des bactéries de type *Leuconostoc mesenteroides.*

d) La dextrane-saccharase de *L. mesenteroides* NRRL B-1299 a la particularité de produire, quant à elle, un dextrane hautement ramifié dont la majorité des ramifications sont de type $\alpha(1\rightarrow2)$. Utilisée en présence de saccharose et de maltose, molécule acceptrice de glucose, elle conduit à la formation de GOS présentant pour certains une liaison $\alpha(1\rightarrow2)$ à leur extrémité non réductrice et pour d'autres des ramifications $\alpha(1\rightarrow2)$ sur les résidus intermédiaires entre les extrémités. A ce titre, ils résistent à la dégradation par les enzymes (hydrolases) du tractus digestif supérieur, chez l'homme et l'animal, et ne sont dégradés que par des genres bactériens capables de fermenter tels *Bacteroides* et *Bifidobacterium*, considérés comme bénéfiques pour l'organisme de l'hôte.

Un phénomène identique se produit au niveau de la peau, permettant d'envisager des applications en cosmétologie, car c'est le déséquilibre de la flore microbienne cutanée qui est à l'origine de nombreux problèmes cosmétiques et dermatologiques. C'est en raison de ces caractéristiques qu'ils sont désignés ici par le terme GOS d'intérêt.

Dans l'ensemble du texte, les polysaccharides synthétisés par les glycosyltransférases ici décrites sont soit des dextranes de haut poids moléculaire lorsque la réaction est réalisée sans accepteur de glucose, soit des oligosides lorsque la réaction est réalisée en présence d'accepteur de glucose tel le maltose ou l'isomaltose sans que cela soit nécessairement spécifié. En effet, la fonctionnalité de l'enzyme est caractérisée par la nature des liaisons glucose-glucose [$\alpha(1\rightarrow6)$, $\alpha(1\rightarrow2)$] ou autres et non par le poids moléculaire du polysaccharide synthétisé.

Les dextrane-saccharases de *L. mesenteroides* trouvent déjà de nombreuses applications dans l'industrie, et en particulier celles de la souche NRRL B-1299 pour lesquelles un procédé de synthèse des GOS présentant des ramifications $\alpha(1\rightarrow2)$ a été décrit dans le brevet EP 0325 872 B1 .

Marguerite Dols *et al.* (2) ont montré que les GOS produits par les dextrane-saccharases de cette souche sont en fait un mélange d'au moins trois familles de molécules similaires différant de fait par le nombre et le positionnement des ramifications de type $\alpha(1\rightarrow2)$, ce qui amène l'hypothèse de l'existence de différentes activités enzymatiques de type glycosyltransférase dans cette souche bactérienne.

Compte tenu de l'intérêt industriel dans le domaine des aliments prébiotiques, en cosmétologie ou en pharmacie des GOS présentant des ramifications $\alpha(1\rightarrow2)$ et rappelé ci-dessus, il a été visé d'isoler et caractériser une enzyme particulière parmi celles produites par *L. mesenteroides* NRRL B-1299 qui serait plus particulièrement impliquée dans la synthèse d'oligosides présentant les ramifications $\alpha(1\rightarrow2)$. L'identification et la caractérisation d'une telle enzyme offrent l'avantage, d'une part, de fournir un procédé de production uniforme et reproductible des GOS d'intérêt et, d'autre part, d'identifier les caractéristiques essentielles de l'enzyme productrice de ces GOS d'intérêt, afin, le cas échéant, d'améliorer les performances des produits de la réaction enzymatique en fonction de l'utilisation envisagée.

Le problème technique sous-tendu par les développements ici décrits était ainsi de pouvoir disposer d'une enzyme et donc des acides nucléiques isolés codant cette enzyme permettant la production améliorée de GOS à ramifications $\alpha(1\rightarrow2)$.

La présente demande apporte une solution technique aux différentes questions évoquées ci-avant en fournissant, selon un aspect de l'invention, une nouvelle dextrane-saccharase, appelée DSR-E codée par un gène doté d'une structure nouvelle et inattendue (*dsr*E) et capable de catalyser la synthèse des glucanes ou des oligosaccharides contenant des ramifications $\alpha(1\rightarrow2)$.

Entre la date de dépôt de la demande de brevet français N°0103631, dont la priorité est revendiquée et dans laquelle la dextrane-saccharase était appelée DSR-D, et celle de la présente demande, une autre dextrane-saccharase, différente de l'enzyme objet de la demande prioritaire, a été décrite et également nommée DSR-D. C'est pourquoi, dans le cadre de la présente description, la dextrane-saccharase décrite, revendiquée dans un mode particulier de réalisation et représentée à la figure 1b) est dénommée non plus DSR-D comme dans le document de priorité, mais DSR-E. De fait, les dextrane-saccharases DSR-D selon ledit document de priorité et DSR-E sont en tout point identiques.

Par structure nouvelle et inattendue, on entend le fait que l'organisation de la protéine diffère de celle de toutes les autres glycosyltransférases décrites à ce jour (1) et dont le domaine catalytique est situé en amont d'un domaine de liaison au glucane, ce dernier constituant la partie carboxylique de la protéine.

Ainsi, la présente demande divulgue un polypeptide isolé ayant une activité enzymatique de glycosyltransférase apte à former des dextranes présentant des ramifications $\alpha(1\rightarrow2)$, caractérisé en ce qu'il comprend au moins un domaine de liaison au glucane et un domaine à activité catalytique situé en aval du domaine de liaison au glucane. Par situé en aval, on entend le fait que la partie aminée de la séquence à activité catalytique ou domaine catalytique est proximale de la partie carboxylique du domaine de liaison au glucane. Ces deux domaines peuvent être immédiatement contigus ou au contraire séparés par une région variable.

La glycosyltransférase selon l'invention comporte de préférence un peptide signal.

Selon l'invention, la glycosyltransférase comprend deux domaines catalytiques situés de part et d'autre du domaine de liaison au glucane.

La présence d'un domaine à activité catalytique dans la partie carboxylique de l'enzyme est une caractéristique essentielle de cette dernière dans sa capacité à former des liaisons $\alpha(1\rightarrow2)$ osidiques. En effet, comme le montrent les expériences décrites ci-après, la délétion de ce domaine dans une enzyme ayant au moins deux domaines catalytiques conduit à la production de glucanes ou d'oligosides ayant essentiellement des liaisons osidiques de type $\alpha(1\rightarrow6)$ et dépourvus de liaisons de type $\alpha(1\rightarrow2)$.

Plus précisément, le domaine catalytique, dès lors qu'il est situé en aval d'un domaine de liaison au glucane, permet la synthèse d'oligosides contenant des liaisons $\alpha(1\rightarrow2)$.

En outre, les expériences décrites ci-après démontrent que la spécificité de fonction de la dextrane-saccharase DSR-E, à savoir sa capacité à catalyser la formation de liaisons osidiques $\alpha(1\rightarrow2)$ est imputable, non pas à la présence concomitante de deux domaines catalytiques, mais plutôt à l'enchaînement d'un domaine de liaison au glucane et d'un domaine catalytique, et plus particulièrement du domaine catalytique CD2.

L'analyse comparative des différentes glycosyltransférases incluant les dextrane-saccharases a mis en évidence un très fort degré de conservation de leur domaine catalytique.

Le domaine catalytique situé dans la partie carboxy-terminale de la glycosyltransférase ici décrite a une séquence présentant au moins 44 % d'identité et 55 % de similarité avec les domaines catalytiques des autres glycosyltransférases analysées. En particulier, le domaine catalytique dans la partie carboxylique de la glycosyltransférase selon l'invention a au moins 65 % d'identité et au moins 80 % de similarité avec la séquence ID No. 1, la triade catalytique Asp/Glu/Asp en positions respectives 230/268/342 étant conservée.

Dans l'ensemble du texte, on entend par X% de similarité par rapport à une séquence de référence le fait que X % des acides aminés sont identiques ou modifiés par substitution conservative telle que définie dans le logiciel d'alignement des séquences d'acides aminés ClustalW (http:///bioweb.pasteur.fr/docs/doc-gensoft/clustalw//) et que (100-X) % peuvent être délétés, substitués par d'autres amino-acides, ou encore que (100-X) % peuvent être ajoutés à la séquence de référence. Une structure primaire particulière de l'enzyme selon la présente description est représentée dans la séquence ID No. 2 qui représente une séquence de 2835 acides aminés d'une dextrane-saccharase de *L. mesenteroides* NRRL B-1299.

Cette dextrane-saccharase, nommée DSR-E, possède comme la plupart des glycosyltransférases et des dextrane-saccharases une séquence signal, une région variable faiblement conservée, un domaine catalytique hautement conservé (CD1), un domaine de liaison au glucane.(GBD) et un deuxième domaine catalytique (CD2) dans la partie carboxylique de la protéine. DSR-E est la première glycosyltransférase analysée et présentant deux domaines catalytiques, dans la configuration présentée dans la figure 1 b). C'est également la première glycosyltransférase dont un domaine catalytique est situé dans la partie carboxylique de la protéine.

La figure 1b) fait apparaître également que le domaine de liaison au glucane est sensiblement plus long que celui décrit précédemment pour les dextranes saccharases connues ; ainsi, une autre caractéristique des enzymes selon l'invention est la taille de ce domaine qui est supérieur à 500 amino-acides.

La comparaison et l'analyse de la séquence de DSR-E avec les séquences des glycosyltransférases ou des dextrane-saccharases déjà décrites (1), ainsi que les moyens utilisés à cette fin sont indiqués dans l'exemple 2 détaillé ci-après. Il y apparaît clairement que si l'existence de deux domaines catalytiques différencie substantiellement DSR-E des autres enzymes, en revanche les séquences desdits domaines sont substantiellement conservées. En particulier, les acides aminés nécessaires à l'activité catalytique sont conservés dans le deuxième domaine catalytique, à savoir la triade Asp/Glu/Asp située aux positions respectives 2210/2248/2322 de la séquence ID No. 2.

La présente invention porte plus particulièrement sur un polypeptide isolé tel que défini en revendication 1.

Selon un autre aspect, la demande divulgue également tout polypeptide isolé ayant une activité catalytique de glycosylstransférase apte à former des dextranes ou des oligosaccharides ayant des ramifications $\alpha(1\rightarrow2)$ tel qu'obtenu par modification, substitution, insertion ou délétion de séquences d'amino-acides mais comportant des séquences présentant au moins 80 % et de préférence au moins 90 % de similarité avec les séquences suivantes de la séquence ID No. 2 :

| | |
|---|---|
| 423 - 439 | 2120 - 2138 |
| 478 - 501 | 2161 - 2184 |
| 519 - 539 | 2202 - 2214 |
| 560 - 571 | 2243 - 2250 |
| 631 - 645 | 2315 - 2322 |
| 1014 - 1021 | 2689 - 2696 |

Selon un aspect particulier, enfin, un polypeptide à activité catalytique contient les acides aminés suivants :

W en positions 425 et 2122,
E en positions 430, 565 et 2127, 2248,
D en positions 487, 489, 527, 638, 2170, 2172, 2210 et 2322,
H en position 637 et 2321,
Q en position 1019 et 2694.

Selon un mode particulier de réalisation, l'invention porte ainsi également sur un polypeptide isolé tel que défini en revendication 2.

Les polypeptides à activité de glycosyltransférases aptes à former des liaisons osidiques $\alpha(1\rightarrow2)$ peuvent se présenter sous forme isolée, ou au contraire intégrés dans une protéine plus large, comme par exemple une protéine de fusion. Il peut être en effet avantageux d'inclure des séquences présentant une autre fonction, comme par exemple une séquence étiquette spécifique d'un ligand permettant d'en faciliter la purification. Ces séquences étiquettes peuvent être du type GST (glutathion-S-Transférase), Intéine - CBD (Chitine-Binding Domaine), (commercialisé par New England Biolabs, http://www.neb.com), MBD (Maltose Binding Domain), polypeptides contenant des résidus histidine contigus permettant de faciliter la purification du polypeptide avec lequel il est fusionné. L'homme du métier peut concevoir toute autre protéine de fusion permettant d'associer la fonction de la DSR-E ici décrite et, selon un aspect particulier, revendiquée, avec une autre fonction, comme par exemple, et sans être limitatif, une séquence augmentant la stabilité de l'enzyme produite par expression dans un hôte recombinant ou une séquence apte à augmenter la spécificité ou l'efficacité d'action de cette enzyme, ou une séquence visant à associer une autre activité enzymatique connexe.

De telles protéines de fusion font également partie de l'invention dès lors qu'elles contiennent le domaine CD2 et le site de liaison au glucane. De la même façon, les fragments de la séquence ID No. 2, comprenant au moins la séquence ID No. 1 et le domaine de liaison au glucane, seuls ou intégrés dans une séquence polypeptidique plus large font partie de l'invention, à partir du moment où l'activité enzymatique de dextrane-saccharase est conservée.

Les variants des séquences polypeptidiques définies ci-dessus font également partie de l'invention. Outre les polypeptides obtenus par substitution conservative des acides aminés telle que définie plus haut, les variants incluent des polypeptides dont l'activité enzymatique est améliorée par exemple par mutagenèse dirigée ou aléatoire, par évolution moléculaire, ou par duplication du domaine catalytique CD2.

La structure particulière de cette enzyme identifiée dans la présente demande résulte d'un processus comprenant :

a) l'identification et l'isolement de la dextrane-saccharase de *L. mesenteroides* catalysant la production des GOS d'intérêt portant les ramifications $\alpha(1\rightarrow2)$ ;
b) le séquençage de fragments de l'enzyme ;
c) la synthèse d'amorces d'amplification aptes à amplifier le gène correspondant de la souche productrice ou des fragments de ceux-ci ;
d) le séquençage des fragments amplifiés ;
e) le clonage dans des vecteurs spécifiques et leur expression dans des hôtes appropriés.

Les modalités du procédé mis en oeuvre sont détaillées dans la partie expérimentale ci-après. La première étape consiste en une séparation des protéines par électrophorèse en gel de polyacrylamide, et identification des bandes présentant l'activité de dextrane saccharase par une réaction enzymatique *in situ* en présence de substrat et d'accepteur. La nature des GOS synthétisés est ensuite identifiée sur chaque bande par analyse HPLC selon les méthodes décrites dans (1). Le temps de rétention des oligosides en HPLC dépend de la nature et de l'organisation de leurs liaisons osidiques. Il est possible en particulier de distinguer ceux constitués de résidus liés en $\alpha(1\rightarrow6)$, en $\alpha(1\rightarrow6)$ avec une ramification $\alpha(1\rightarrow2)$ à l'extrémité non réductrice de la molécule, et ceux recherchés composés d'une chaîne linéaire $\alpha(1\rightarrow6)$ avec des ramifications $\alpha(1\rightarrow2)$.

Les inventeurs ont donc isolé et identifié la dextrane-saccharase de *L. mesenteroides* NRRL B-1299 productrice des GOS d'intérêt.

Un procédé d'ingénierie reverse mis en oeuvre dans les étapes b) à e) ci-dessus a permis ensuite de fournir la séquence nucléotidique codant l'enzyme et permettant de la produire en quantité industrielle et le cas échéant de la modifier, d'en améliorer ses performances par les techniques à la disposition de l'homme du métier. A titre d'exemple, on peut citer la mutagénèse dirigée ou aléatoire, ou l'évolution moléculaire (DNA shuffling) (3).

Un autre aspect de la présente demande porte sur une molécule d'acide nucléique isolée codant une enzyme à activité glycosyltransférase apte à former des dextranes ou des oligosides présentant des ramifications $\alpha(1\rightarrow2)$ et comprenant au moins une séquence codant un domaine de liaison au glucane, et au moins une séquence nucléotidique codant un domaine catalytique situé en 3' de la précédente, ladite séquence codant un domaine catalytique ayant au moins 50 % et de préférence au moins 70 % de similarité avec la séquence ID No. 3 ayant la structure présentée en figure 1b.

Par similarité, on entend le fait que, pour un même cadre de lecture, un triplet donné est traduit par le même acide aminé. Ce terme inclut donc les modifications de bases résultant de la dégénérescence du code génétique.

Le pourcentage de similarité est déterminé en comparant une séquence donnée avec la séquence de référence. Lorsque celles-ci sont de longueurs différentes, le pourcentage de similarité est basé sur le pourcentage de nucléotides de la séquence la plus courte similaires à ceux de la séquence la plus longue.

Le degré de similarité peut être déterminé conventionnellement par utilisation de logiciels tels le ClustalW (Thompson et al., Nucleic Acid Research (1994), 22 4673-4680) distribués par Julie Thompson (Thompson@EMBL-Heidelberg.DE) et Toby Gibson (Gibson@EMBL-Heidelberg.DE) du Laboratoire Européen de Biologie Moléculaire, Meyerhosfstrasse 1, D-69117 Heidelberg, Germany. ClustalW peut aussi être chargé à partir de plusieurs sites web incluant IGBMC (Institut de Génétique et de Biologie Moléculaire et Cellulaire, B.P. 163, 67404 Illkirch cedex France; ftp://ftp-igbmc.u-strabg.fr/pub/) et EBI (ftp://ftp.ebi.ac.uk/pub/software/) et tous les sites renvoyant à l'Institut de Bioinformatique (Wellcome Trust Genome Campus, Hinxton, Cambridge CB10 1SD, UK).

Les acides nucléiques isolés ici décrits peuvent comprendre notamment d'autres séquences destinées à améliorer l'expression et/ou l'activité de l'enzyme produite.

Il peut s'agir à titre d'exemple :

- des séquences codant une séquence signal pour leur sécrétion ;
- une duplication de la séquence codant le domaine catalytique CD2.

De façon préférée, un acide nucléique isolé selon l'invention comprend :

a) deux séquences codant des domaines catalytiques ayant au moins 50 %, et de préférence au moins 80 % de similarité avec la séquence ID n° 3 ayant la structure présentée en figure 1b;
b) une séquence codant le domaine de liaison au glucane, cette dernière étant située de préférence entre les deux séquences en a).

Un acide nucléique tel qu'ici décrit pourra comprendre en outre :

- un promoteur, apte à son expression dans une cellule hôte choisie,
- une séquence codant un peptide signal, et/ou
- une ou des séquences variables,

cette ou ces séquence(s) étant toutes situées en partie 5' des séquences codant le ou les domaine(s) catalytique(s).

Un exemple particulier d'un acide nucléique isolé comprend plus particulièrement :

a) la séquence ID No. 4,
b) une séquence présentant au moins 80 % de similarité avec la séquence ID n° 4 ayant la structure présentée en figure 1b, ou
c) le brin complémentaire de la séquence a) ou b), ou
d) une séquence hybridant a), b) ou c).

L'hybridation en d) est réalisée en conditions standard, et de préférence en conditions stringentes. Par hybridation en condition stringente, on entend le fait qu'il existe une identité de séquences d'au moins 80 % de la séquence que l'on cherche à hybrider et de préférence une identité d'au moins 90 % de la séquence que l'on cherche à hybrider, dans des conditions décrites par exemple dans Sambrook et Russel (3ème édition, 2001, Cold Spring Harbour Laboratory Press, Cold Spring Harbour, NY).

L'invention porte plus particulièrement sur un acide nucléique isolé tel que défini en revendications 3 et 4.

La demande porte également sur un gène codant une dextrane-saccharase apte à former au moins 15 % de ramifications α(1→2). Outre la séquence codante, le gène comprend les séquences permettant l'initiation de la transcription ainsi que les séquences permettant l'attachement de l'ARN messager au ribosome (RBS). La séquence ID No. 5 représente une structure du gène tel qu'isolé de *L. mesenteroides* NRRL B-1299.

Les nucléotides en amont de l'ATG d'initiation de la traduction sont numérotés 1 à 232.

On peut identifier l'existence d'une séquence RBS entre les nucléotides 218 et 223, ainsi que les séquences consensus - 35 et - 10 situées entre les nucléotides 82 et 86 (TTGAA), d'une part et 100 et 105 (ATAAAT), d'autre part.

Toute séquence d'acide nucléique hybridable avec l'ADN de la séquence ID No. 4 ou son brin complémentaire est susceptible de coder une enzyme ayant les propriétés et caractéristiques de l'enzyme ici décrite. Ceci s'applique tant aux séquences naturelles existant dans d'autres microorganismes que *L. mesenteroides* NRRL B-1299 et isolées de banques génomiques de microorganismes, que celles préparées par génie génétique ou par synthèse chimique.

En particulier, les séquences en amont de l'ATG d'initiation de la traduction et nécessaires à l'expression de la protéine peuvent être avantageusement substituées par des séquences d'initiation de la transcription et/ou de fixation au ribosome adaptés au système d'expression choisi pour la séquence codante.

Une séquence d'acides nucléiques susceptible de s'hybrider en condition stringente avec l'acide nucléique isolé selon la présente demande comprend également des fragments, des dérivés, ou des variants alléliques de la séquence d'acides nucléiques de la présente demande qui code une protéine ayant l'activité enzymatique décrite ci-avant. Ainsi, les fragments sont définis comme des fragments de molécules d'acides nucléiques suffisamment longs pour coder une protéine ayant conservé son activité enzymatique. Celle-ci inclut aussi bien des fragments dépourvus de la séquence codant le peptide signal responsable de la sécrétion de la protéine.

Le terme "dérivé" signifie séquence, différente de la séquence originelle, à une ou plusieurs positions, mais présentant un haut degré de similarité avec ces séquences. Dans ce contexte, similarité signifie une identité d'au moins 80 % des nucléotides, et de préférence d'au moins 90 % avec la séquence originelle. Les modifications dans ce cas portent sur des délétions, substitutions, insertions ou recombinaisons, à partir du moment où l'enzyme codée par ces séquences homologues présentent l'activité enzymatique des polypeptides ici décrits.

Les séquences d'acides nucléiques selon la présente demande telles que décrites ci-dessus et qualifiées de dérivés de ces molécules telles que définies ci-avant, sont généralement des variants exerçant la même fonction biologique. Ces variations peuvent être des variations naturelles, notamment celles observables d'une espèce à l'autre et résultant d'une variabilité inter espèce ou au contraire être introduites par le moyen d'une mutagenèse dirigée, aléatoire ou par DNA Shuffling (évolution moléculaire).

De la même façon, font partie de la présente divulgation les acides nucléiques isolés codant une glycosyltransférase apte à catalyser la synthèse de dextrane ou d'oligosaccharide portant au moins 20 % et de préférence au moins 30 % de ramifications de type α(1→2) et obtenus par évolution moléculaire (DNA shuffling) et comprenant :

- une étape de modification aléatoire d'une des séquences décrites précédemment et, en particulier, des séquences ID Nos. 3 et 4 et d'établissement de variants ;
- une étape d'expression de ces séquences modifiées dans une cellule hôte appropriée, un hôte abritant un variant ;
- une étape de criblage des hôtes exprimant une enzyme apte à former plus de 20 % et de préférence plus de 30 % de liaisons α(1 → 2) sur un substrat approprié et une étape d'isolement du ou des gènes améliorés.

Selon un autre aspect de l'invention, cette dernière porte également sur un procédé tel que défini en revendication 11.

Un acide nucléique isolé selon la présente demande pourra également comprendre :

a) une séquence ayant au moins 80 % de similarité avec la séquence codant une dextrane-saccharase exprimée par le plasmide pCR-T7-*dsr*E dans E. *coli* déposé à la CNCM le 15 mars 2001 sous le numéro I-2649 (E. *coli* JM 109 [pCR-T7-*dsr*iD]), ou

b) une séquence complémentaire de la séquence en a).

La dénomination de la souche transformée par le plasmide recombinant pCR-T7-*dsr*E et déposée à la CNCM est celle indiquée ci-dessus entre parenthèses. Cela n'affecte pas le changement de dénomination du gène opéré ultérieurement au dépôt de ladite souche pour les raisons évoquées supra.

La demande divulgue également les fragments d'acides nucléiques tels que définis ci-dessus, hybridables avec la séquence ID No. 4, et utilisables comme sondes d'hybridation pour la détection de séquences codant des enzymes ici décrites. Ces fragments peuvent être préparés par toutes les techniques connues de l'homme du métier.

Outre les sondes d'hybridation, des amorces d'amplification font également partie de la présente demande. Lesdites amorces sont des fragments hybridables avec la SEQ ID No. 4 ou avec son brin complémentaire et permettent l'amplification de séquences spécifiques codant des dextrane-saccharases présentes dans un organisme procaryote ou eucaryote, animal ou végétal.

L'utilisation de telles amorces d'amplification permet la mise en oeuvre d'un procédé d'identification de l'existence éventuelle d'un gène codant une enzyme apte à catalyser la synthèse de GOS avec des ramifications $\alpha(1\rightarrow2)$ dans un tel organisme, ledit procédé faisant également partie de la présente divulgation.

Sont également divulgués des vecteurs d'expression comprenant un acide nucléique tel que décrit ci-avant, sous le contrôle de séquence permettant son expression et de préférence son excrétion dans des cellules procaryotes ou eucaryotes. Par cellules procaryotes, on choisira de préférence des bactéries choisies dans un groupe comprenant E. *coli,* les *Lactococcus*, les *Bacillus*, les *Leuconostoc*. Par cellules eucaryotes, on choisira de préférence les eucaryotes choisis dans un groupe contenant les levures, les champignons ou les végétaux.

Le vecteur comprend un promoteur adapté à l'expression de l'acide nucléique isolé selon la présente description dans le système d'expression choisi. A titre d'exemple, le promoteur du bactériophage T7 pourrait être avantageusement choisi pour une expression dans E. *coli.* L'invention porte plus particulièrement sur un vecteur tel que défini en revendication 5 ou 6.

La demande divulgue également des cellules hôtes, procaryotes ou eucaryotes, transformées par un acide nucléique tel qu'ici décrit de préférence compris dans un vecteur d'expression portant un promoteur, adapté à une expression dans les cellules hôtes choisies. Les cellules transformées sont choisies dans le groupe des bactéries à Gram- telle E. *coli,* ou dans le groupe des bactéries à Gram+ telles *Lactococcus*, *Bacillus*, *Leuconostoc* ou parmi les eucaryotes dans un groupe comprenant les levures ou les champignons, ou les végétaux.

Un exemple particulier d'une cellule transformée selon la présente description est la souche E. *coli* hébergeant un plasmide appelé PCR-T7*dsr*E et porteur de la séquence ID No. 4 sous le contrôle du promoteur du bactériophage T7 et déposée à la CNCM le 15 mars 2001 sous le numéro I-2649. L'invention porte plus particulièrement sur des cellules telle que définies en revendications 7 ou 8.

La présente demande, par ailleurs, divulgue un procédé de production d'une glycosyltransférase apte à former des dextranes ou des oligosides présentant au moins 15 % et de préférence au moins 20 % de ramifications de type $\alpha(1\rightarrow2)$ osidiques et comprenant :

a) l'insertion d'un acide nucléique ou d'un vecteur tel que décrit précédemment dans une cellule hôte apte à l'exprimer et de préférence à sécréter la glycosyltransférase ;
b) la caractérisation de l'activité enzymatique recherchée par toutes les méthodes accessibles à l'homme du métier ;
c) la purification de l'enzyme à partir d'un extrait cellulaire.

Selon d'autres aspects de l'invention, cette dernière porte également sur un procédé tel que défini en revendication 9 ou 10.

Par méthode de caractérisation de l'activité enzymatique connue de l'homme du métier, on comprendra les méthodes décrites dans la littérature par exemple dans la référence (2) ainsi que de nouvelles méthodes susceptibles d'être mises au point permettant d'identifier et de discriminer les glucooligosaccharides présentant le taux de ramification recherché.

Il s'agit en fait de tout procédé de criblage permettant d'identifier la présence de ramifications $\alpha(1\rightarrow2)$ dans un GOS.

A titre d'exemple, seront utilisées :

- l'HPLC pour lequel la migration des GOS varie en fonction de la nature et le positionnement des ramifications, notamment ceux ayant le lien $\alpha(1\rightarrow2)$ à l'extrémité réductrice et ceux ayant ce lien sur l'avant-dernier glucose, et/ou
- la Résonance magnétique nucléaire (RMN),
- l'existence d'une réaction positive avec des anticorps monoclonaux spécifiques des liaisons $\alpha(1\rightarrow2)$ sur l'extrémité réductrice et/ou d'anticorps monoclonaux spécifiques des liaisons $\alpha(1\rightarrow2)$ sur l'avant-dernier glucose du GOS.

La demande divulgue également un procédé d'obtention d'une glycosyltransférase apte à présenter des oligosides ou des dextranes présentant un taux de ramification $\alpha(1\rightarrow2)$ supérieur à 15 % et de préférence supérieur à 30 % de la totalité des liaisons osidiques et comprenant une étape de modification de la séquence ID No. 4 par addition, délétion, mutation à partir du moment où :

- le cadre de lecture n'est pas modifié, et
- les acides aminés suivants sont conservés après traduction :

W en positions 425 ou 2122, codé par le triplet TGG en positions 1273 et 6364,
E en positions 430, 565, 2127 et 2248 codés par les triplets GAA en positions 1288, 1693, 6379 et 6742 respectivement,

D en positions 487, 489, 527, 638, 2170 et 2210 codés par les triplets GAT en positions 1459, 1465, 1579, 1912, 6508 et 6628 respectivement,
D en positions 2172 et 2322 codés par les triplets GAT en positions 6514 et 6964,
H en position 637 et 2321, codés respectivement par les triplets CAT en position 1909 et CAC en position 6961,
Q en positions 1019 et 2694 codés respectivement par les triplets CAA (position 3055) et CAG (position 8080).

Un procédé de production d'une glycosyltransférase selon la présente demande ayant les mêmes caractéristiques que ci-avant peut également comprendre :

- une étape de modification aléatoire de la séquence ID n° 4 et d'établissement d'une banque de variants,
- une étape d'expression de ces séquences modifiées dans une cellule hôte appropriée, un hôte abritant un variant,
- une étape de criblage des hôtes exprimant une enzyme apte à former plus de 15% et de préférence plus de 30% de liaison $\alpha(1 \rightarrow 2)$ sur un substrat approprié,
- une étape d'isolement du ou des gènes améliorés.

Dans un autre mode de réalisation, le procédé consiste à modifier la séquence ID No. 3 par duplication de tout ou partie du domaine catalytique CD2.

On pourra comprendre que les procédés ci-dessus visent non seulement à l'obtention d'une glycosyltransférase apte à former des oligosides présentant un taux de ramification $\alpha(1 \rightarrow 2)$ constant et reproductible, supérieur à 15% des ramifications totales mais également à améliorer le taux de ramification $\alpha(1 \rightarrow 2)$ dans l'objectif de modifier les propriétés des oligosides obtenus dans le sens d'une amélioration de leurs propriétés diététiques ou de leur capacité à maintenir ou rétablir la flore bactérienne associée à certains organes du corps humain ou animal.

La présente demande divulgue enfin des glycosyltransférases susceptibles d'être obtenues par un procédé cité ci-avant et apte à former au moins 15 % et de préférence au moins 30 % de ramifications de type $\alpha(1 \rightarrow 2)$ osidiques dans des glucooligosaccharides.

La demande divulgue enfin l'utilisation des glycosyltransférases ici décrites ainsi que celles susceptibles d'être obtenues par les procédés ci-dessus, dans la fabrication d'une composition à effet prébiotique ou dans la fabrication d'une composition dermatologique, cosmétique ou pharmaceutique. L'invention porte plus particulièrement sur l'utilisation définie en revendication 12.

A titre d'exemples non limitatifs, on peut citer l'amélioration du transit intestinal chez les animaux et chez l'homme, l'amélioration de l'assimilation du calcium et/ou du magnésium et des minéraux en général, la prévention du cancer du côlon, la prévention ou le traitement des affections de la peau telles l'acné, les pellicules, les odeurs corporelles.

L'avantage des polypeptides et des acides nucléiques codant ces polypeptides ici décrits ou selon l'invention se situe non seulement au niveau de l'amélioration en terme de qualité, de rendement, de reproductibilité, et de prix de revient des glycosyltransférases aptes à former des oligosaccharides avec des ramifications de type $\alpha(1 \rightarrow 2)$ osidiques mais également dans la perspective de produire de nouvelles enzymes dont la fonctionnalité est améliorée.

Les figures, exemples et description détaillés ci-après permettent, sans la limiter, d'illustrer les caractéristiques et les fonctionnalités particulières des polypeptides à activité enzymatique et des séquences codant ceux-ci. Elles permettent en particulier d'illustrer de façon plus précise la spécificité du domaine catalytique présent dans la partie carboxylique de l'enzyme ici décrite ou selon l'invention, et son évolution potentielle pour l'obtention d'enzymes améliorées.

**LEGENDE DES FIGURES** :

Figure 1 : structure des glycosyltransférases natives et des protéines recombinantes dérivées : la figure 1a) représente la structure des glycosyltransférases et des dextrane-saccharases décrites dans la littérature (1). PS : peptide signal ; ZV: région variable, CD : domaine catalytique, GBD : domaine de liaison au glucane. La figure 1b) représente la structure de la glycosyltransférase selon l'invention. Les figures 1c) à 1i) représentent différentes constructions comportant des délétions en comparaison de la protéine DSR-E native. $\Delta$(PS) correspond au contrôle constitué par la forme entière clonée dans le système pBAD-TOPO Thiofusion (Invitrogen).

Figure 2 : schéma récapitulatif de la méthode de clonage de la séquence nucléotidique codant une glycosyltransférase selon la présente demande à l'aide d'une bibliothèque génomique en utilisant une sonde PCR décrite dans le tableau I et une sonde *Hind*III/EcoRV respectivement.

Figure 3 : comparaison des séquences signal de différentes glycosyltransférases de L. mesenteroides. Les acides aminés conservés sont en gras. DSR-B : *L. mesenteroides* NRRL B-1299 (4) ; DSR-S : *L. mesenteroides* NRRL B-512F (5) ; ASR: *L. mesenteroides* NRRL B-1355 (6).

Figure 4 : alignement des 11 séquences répétées de l'enzyme DSR-E et observées dans la zone variable.
Figure 5 : alignement des séquences conservées du domaine catalytique.

- Bloc A : acides aminés essentiels de la partie N-terminale du domaine catalytique ;
- Bloc B : acides aminés de la partie du domaine catalytique de liaison au saccharose ;
- Blocs C, D, E : blocs contenant les trois résidus d'acides aminés impliqués dans la triade catalytique (6) ;
- Bloc F : séquence contenant la glutamine 937 de GTF-I étudiée par Monchois *et al.* (7).

Les acides aminés entièrement conservés sont indiqués en gras. « * » : substitutions conservatives ; « : » : substitutions semi-conservatives ; --- : GAP. Les numérotations sont celles de la séquence ID No. 2.
Figure 6 : caractérisation HPLC des produits synthétisés par l'enzyme recombinante DSR-E.

6A : analyse en HPLC des glucooligosaccharides obtenus avec les dextrane-saccharases de *L. mesenteroides* NRRL B-1299.
6B : analyse HPLC des glucooligosaccharides obtenus par la DSR-E recombinante. L'identification des différents pics suivants :

1 : fructose,
2 : maltose,
3 : sucrose,
4 : panose,
5 : R4,
6 : OD4,
7 : R5,
8 : OD5,

A, B, C : pics non identifiés.
6C : DSR-E recombinante délétée du domaine catalytique de la partie carboxylique de l'enzyme ($\Delta$ DSR-E).

Figure 7 : analyse HPLC des produits de la réaction d'accepteur sur maltose, synthétisés par les différentes formes entières et délétées de la protéine DSR-E.

L. m. B-1299 : mélange de dextrane-saccharases produit par *L. mesenteroides* NRRL B-1299.
L'identification des différents pics se fait comme suit :

F : fructose
M : maltose
S : saccharose
P : panose
R4, R5 : GOS comportant des liaisons $\alpha(1\rightarrow2)$
OD4, OD5 : GOS dépourvus de liaisons $\alpha(1\rightarrow2)$.

**MATERIELS ET METHODES :**

1) Souches bactériennes, plasmides et conditions de croissance :

Toutes les souches sont conservées à -80°C dans des tubes contenant 15% de g!ycérol (v/v).
*Leuconostoc mesenteroides* B-1299 (NRRL, Peoria, USA) est cultivée à 27°C, sous agitation (200 RPM) sur milieu standard (saccharose 40 $g.l^{-1}$, phosphate potassium 20 $g.l^{-1}$, extrait de levure 20 $g.l^{-1}$, $MgSO_4$-$7H_2O$ 0.2 $g.l^{-1}$, $MnSO_4$-$H_2O$ 0.01$g.l^{-1}$, NaCl 0.01$g.l^{-1}$, $CaCl_2$ 0.02 $g.l^{-1}$, $FeSO_4$-$7H_2O$ 0.01 $g.l^{-1}$), le pH étant ajusté à 6,9.
*Escherichia coli* DH5$\alpha$ et JM109 ont été cultivées sur milieu LB (Luria-Bertani).
La sélection des clones recombinants de pUC18 ou pGEM-T Easy est effectuée sur boites LB-agar supplémenté avec 100 $\mu g.ml^{-1}$ d'ampicilline, 0.5 mM d'isopropyl-$\beta$-D-thiogalactopyranoside (IPTG) et 40 $\mu g.ml^{-1}$ de 5-bromo-4-chloro-3-indolyl-$\beta$-D-galactopyranoside (X-gal). Des cellules d' *E. coli* TOP 10 ont été utilisées pour le système de clonage de produit PCR TOPO Cloning (Invitrogen), et cultivées sur milieu LB supplémenté de kanamycine à la concentration de 50 $\mu g.ml^{-1}$.
En ce qui concerne l'expression de *dsrE,* le kit de clonage ECHO Cloning System (Invitrogen) permet le clonage d'un produit PCR dans un vecteur donneur (pUNI/V5-His-TOPO), précédant une étape de recombinaison avec un vecteur accepteur adapté (pCR-T7-E). Ce système requiert des cellules *E. coli* PYR1, TOP 10 et BL21(DE3)pLysS cultivées sur milieu LB supplémenté de 50 $\mu$g.ml$^{-1}$ de kanamycine, ainsi que de 34 $\mu$g.ml$^{-1}$ de chloramphénicol pour la souche BL21(DE3)pLysS.

Les plasmides pUC18 digérés et déphosphorylés proviennent de Pharmacia (Amersham Pharmacia Biotech) et ont été utilisés pour la constitution de banque d'ADN génomique de *L. mesenteroides* NRRL B-1299. Le clonage de produit PCR a, quant à lui, nécessité l'emploi de plasmide pGEM-T Easy (Promega), et pour les fragments de plus de 2 kbp, de plasmide TOPO-XL (Invitrogen).

Le système pBAD-TOPO Thiofusion (Invitrogen), utilisé pour construire les différentes formes délétées de la protéine DSR-E, met en jeu le promoteur araBAD, dont les mécanismes de contrôle impliquent la protéine régulatrice AraC. En l'absence d'inducteur, à savoir le L-arabinose, la protéine dimérique AraC s'associe aux structures régulatrices de l'opéron et entraîne la formation d'une boucle d'ADN, ladite boucle bloquant ainsi la transcription des gènes placés sous le contrôle du promoteur araBAD. En présence de L-arabinose en revanche, AraC forme un complexe, ce qui libère la boucle d'ADN et permet l'initiation de la transcription. L'expression basale peut être limitée en ajoutant du glucose au milieu de culture : celui-ci intervient en diminuant le niveau d'AMP cyclique, et donc l'activation concomitante de la protéine CAP (cAMP activator protein). Le niveau d'activation obtenu est fonction de la concentration en L-arabinose, de telle sorte que les conditions optimales de production de la protéine d'intérêt peuvent être sélectionnées avec précision.

De plus, l'emploi de ce vecteur permet de positionner une étiquette thioredoxine de 12 kDa du côté N-terminal de la protéine d'intérêt. Cette fusion vise à favoriser la traduction du gène codant ladite protéine d'intérêt. La protéine étiquette permet en outre d'augmenter la solubilité de la protéine à laquelle elle se trouve fusionnée. Le système pBAD-TOPO Thiofusion est conçu pour permettre d'éliminer facilement l'étiquette thioredoxine, par simple clivage à l'aide de l'entérokinase. Enfin, grâce à ce système d'expression, une étiquette histidine est insérée du coté de l'extrémité C-terminale de la protéine d'intérêt. Une telle étiquette est utile pour purifier ladite protéine par affinité.

Dans le cadre de l'utilisation de ce système, la souche *E. coli* TOP 10 a été cultivée sur milieu LB supplémenté de 100 $\mu$g.ml$^{-1}$ d'ampicilline.

2) Electrophorèse sur gel, localisation et caractérisation de l'enzyme :

Après une culture de *L. mesenteroides* NRRL B-1299 de 7h, le milieu a été centrifugé (7000 RPM, 4°C, 30 min) et les cellules, où 90% de l'activité enzymatique se retrouve, ont été concentrées 10 fois dans une solution de tampon acétate (20 mM, pH 5,4), chauffées 5 minutes à 95°C en présence de solution de dénaturation (Tris HCI 62.5 mM, SDS 4%, urée 6M, bleu de bromophenol 0.01% et $\beta$-mercaptoethanol 200 mM). 300 $\mu$l du mélange a été déposé sur gel de polyacrylamide à 7%. Après migration, les protéines totales ont été révélées par coloration au noir amido, alors que l'activité dextrane-saccharase a été détectée par coloration du polymère au réactif de Schiff après synthèse de dextrane *in situ.* Les bandes correspondant à des dextrane-saccharases actives ont été excisées et incubées séparément dans 2 ml de solution d'acétate de sodium 20 mM pH 5.4 contenant 100g.l$^{-1}$ de saccharose et 50 g.l$^{-1}$ de maltose. Après consommation totale du saccharose, la réaction a été arrêtée par chauffage à 95°C pendant 5 minutes, et le milieu réactionnel centrifugé 5 minutes à 15000g afin d'éliminer le dextrane insoluble. Les échantillons ont été analysés par chromatographie en phase inverse (colonne C18, Ultrasep 100, 6 $\mu$m, 5x300mm, Bishoff Chromatography) en utilisant de l'eau ultrapure comme éluant, à un débit constant de 0.5 ml.min$^{-1}$. Les oligosaccharides ont été séparés pendant 30 minutes à température ambiante, et détectés par réfractométrie. Le séquençage peptidique a été réalisé sur les bandes protéiques sélectionnées par le Laboratoire de Microséquençage, Institut Pasteur, Paris.

3) Techniques de biologie moléculaire utilisées :

La purification du plasmide de E. *coli* et la purification de l'ADN génomique de *L. mesenteroides* ont été réalisées en utilisation respectivement QiaPrep Spin Plasmid kit et le Cell Culture DNA maxi kit (QiaGen). Les procédés d'amplification et de clonage ont été réalisés en utilisant les techniques standard (Sambrook et Russel, 2001, supra). Les enzymes de restriction et de modification, provenant des sociétés commerciales New England Biolabs ou Gibco BRL, ont été utilisées selon les protocoles des fabricants.

La PCR a été réalisée avec des amorces choisies sur la base de la séquence protéique obtenue sur une bande de gel d'électrophorèse isolée (voir supra, électrophorèse sur gel et localisation de l'enzyme). Deux peptides ont été sélectionnés :

- 29-FYFESGK, et
- 24-FESQNNNP

et utilisés pour synthétiser des oligonucléotides dégénérés et indiqués dans le tableau I ci-dessous.

Dans ce tableau où les numérotations sont celles de la séquence ID no. 4, il apparaît que la présence d'un résidu sérine dans les deux peptides nécessite la synthèse de deux amorces pour chaque peptide dans la mesure où la sérine peut être codée par six codons différents. ECHO-dir et ECHO-inv sont les amorces utilisées ayant permis l'amplification de *dsr*E par PCR pour son clonage dans le système d'expression ECHO Cloning (Invitrogen).

**TABLEAU I** :

| Désignation | Description | Séquence 5'-3' |
|---|---|---|
| 29-dir1 | **FYFESGK** | TT(C/T)TA(C/T)TT(C/T)GA(A/G)*TCA*GG(C/G)AA(A/G) |
| 29-dir2 | | TT(C/T)TA(C/T)TT(C/T)GA(A/G)*AGC*GG(C/G)AA(A/G) |
| 24-inv1 | **FESQNNNP** | (T/G)GG(G/A)TT(G/A)TT(G/A)TTTTG*TGA*(T/C)TCAAA |
| 24-inv2 | | (T/G)GG(G/A)TT(G/A)TT(G/A)TTTTG*GCT*(T/C)TCAAA |
| IPCR-rev | séquence nt 5769-5798 | CCCTTTACAAGCTGATTTTGCTTATCTGCG |
| IPCR-dir | séquence nt 8311-8342 | GGGTCAAATCCTTACTATACATTGTCACACGG |
| ECHO-dir | séquence nt -6 - 39 | AGTTGT**ATG**AGAGACATGAGGGTAATTTGTGACCGTAAAAAATTG |
| ECHO-inv | séquence nt 8457-8504 | ATTTGAGGTAATGTTGATTTATCACCATCAAGCTTGAAATATTGACC |

**PCR** :

La PCR a été réalisée en utilisant un thermocycleur Perkin-Elmer, modèle 2400, et avec 50 nanogrammes d'ADN génomique. Les quantités d'amorces utilisées étaient de 10 μM de 29-Dir1 et de 24-Inv1. Au mélange réactionnel, ont été ajoutés 250 μM de chaque désoxynucléotide triphosphate, et la Taq Polymérase.

Après une amplification de 25 cycles à 94° C pendant 30 secondes puis à 50° C pendant 30 secondes, puis à 72° C pendant 5 minutes, un fragment de 666 paires de base a été obtenu.

Certains fragments ont été amplifiés à l'aide du système « Expand Long Template PCR » (Roche Boehringer Mannheim), conformément aux indications d'utilisation du fournisseur. Ce système permet d'amplifier des fragments de grande taille, jusqu'à environ 20 kbp, de manière très efficace. La combinaison de deux ADN polymérases permet notamment de minimiser le taux d'erreur durant les étapes d'élongation.

**Hybridation southern et bibliothèque génomique de L. *mesenteroides* NRRL B-1299** :

L'ADN chromosomique de *L. mesenteroides* NRRL B-1299 a été digéré avec différentes enzymes de restriction, puis séparé par électrophorèse sur gel d'agarose à 0,8 % en tampon TAE 0,5X.

Des bibliothèques génomiques de la bactérie ont été transférées sur des membranes de nylon hybond N+ (Amersham PharmaciaBiotech). L'hybridation a été réalisée en utilisant le fragment de 666 paires de bases à la désoxy-adénosine-triphosphate marqué au $^{32}P$. La réaction de marquage a été réalisée en utilisant le kit de marquage "Mega Prime DNA Labelling System Kit" (Amersham PharmaciaBiotech), suivie par la purification de la sonde sur des colonnes MicroSpin S-200HR. La pré-hybridation et l'hybridation ont été réalisées en conditions fortement stringentes (65° C pendant la nuit, selon les méthodes habituelles) (Sambrook et Russel, 2001, supra).

**PCR inverse** :

La réaction de PCR inverse permet d'obtenir un fragment d'ADN linéaire à partir d'une matrice circulaire en utilisant des amorces divergentes.

L'ADN génomique de *L. mesenteroides* NRRL B-1299 a été digéré par EcoRV dans les conditions recommandées par le fournisseur.

Après re-circularisation, les produits de digestion ont été utilisés comme matrice dans une réaction de PCR inverse [Extrapol II DNA polymerase (Eurobio), volume réactionnel de 50 μl, paramètres de la réaction de PCR inverse : 25 cycles ; 94° C, 30 secondes ; 51° C, 30 secondes ; 72° C, 3 minutes]. Les deux amorces ont été choisies en fonction de la séquence de l'insert de pSB2 comme indiqué dans la figure 2.

La figure 2 résume les modalités d'obtention des différents plasmides porteurs des fragments de *dsr*E par criblage de la bibliothèque génomique et utilisation des sondes décrites ci-dessus.

**Séquence d'ADN et analyse** :

Après le séquençage des peptides, des amorces dégénérées dessinées en tenant compte de la fréquence d'utilisation des codons dans les gènes de dextrane-saccharases *de L. mesenteroides* NRRL B-1299, ont été synthétisées et ont permis l'amplification d'un fragment de 666 bp. Le séquençage de ce fragment a révélé de fortes homologies avec les gènes de dextrane-saccharases déjà connus, tout en étant totalement nouveau.

L'utilisation de ce fragment comme sonde homologue dans des expériences de Southern, a permis de repérer des signaux positifs sur différentes pistes d'ADN génomique digéré. Une première banque *Hind*III a ainsi été criblée, et un plasmide recombinant, nommé pSB2, contenant un insert de 5,6 kbp, a été purifié. L'analyse de la séquence de ce fragment *Hind*III a révélé un cadre ouvert de lecture couvrant la totalité de l'insert. Ensuite, une banque *Eco*RV a été criblée avec une sonde *Hind*III/*Eco*RV isolée à l'extrémité N-terminale de l'insert *Hind*III de 5,6 kbp. Un plasmide recombinant pSB3, testé positivement par dot-blot, s'est avéré contenir un insert de 3,8 kbp qui, après séquençage, a été montré contenir le codon d'initiation de la traduction et la région promotrice du nouveau gène de dextrane-saccharase nommé *dsr*E.

Dans le but d'obtenir le codon de terminaison de *dsr*E, une PCR inverse a été réalisée sur de l'ADN génomique de *L. mesenteroides* NRRL B-1299 digéré par *Eco*RV et religué sur lui-même, en utilisant des amorces oligonucléotidiques divergentes dessinées à partir de la séquence de l'insert pSB2. Un fragment unique à la taille attendue de 1 kbp a été amplifié puis cloné dans un pGEM-T Easy, pour obtenir le plasmide pSB4. Après séquençage, la séquence amplifiée située en aval du site *Hind*III comporte 221 bp et contient le codon de terminaison du cadre de lecture de *dsr*E, situé 30 bp en aval du site de restriction *Hind*III.

Le séquençage des différents fragments portés par les trois plasmides a été réalisé par la société Génome Express et ce, sur les deux brins. Les analyses des séquences de nucléotides ont été réalisées en utilisant le "ORF Finder" (http://www.ncbi.nlm.nih.gov/gorf/gorf.html), Blast (http://www.ncbi.nlm.nih.gov/blast/blast.cgi, Altschul *et al.,* 1997) ClustalW (http://www2.ebi.ac.uk/clustalw, Thompson *et al.,* 1994), PRODOM (http://protein.toulouse.inra.fr/prodom.html, Corpet *et al.,* 2000), PFAM (http://pfam.wustl.edu/hmmsearch.shtml, Bateman *et al.,* 2000) et SAPS (http://bioweb.pasteur.fr/seqanal/interfaces/saps.html, Brendel *et al.,* 1992), l'ensemble de ces logiciels étant accessible par internet.

**Expression de la protéine** :

Deux systèmes de clonage et d'expression ont été utilisés aux fins de la production de protéines recombinantes chez *E. coli,* à savoir les systèmes ECHO-Cloning et pBAD-TOPO Thiofusion (Invitrogen).

A titre d'exemple, se trouve brièvement décrite ci-après la méthode de clonage de la séquence nucléotidique codant la protéine DSR-E au moyen du système ECHO-Cloning.

Deux amorces telles que proposées dans le tableau I ci-dessus ont été utilisées dans le cadre de l'amplification à l'aide du système "Expand Long Template" dans les conditions suivantes : 94° C pendant 3 minutes suivis de 25 cycles à 94° C pendant 30 secondes, 55° C pendant 30 secondes, et 68° C pendant 7 minutes. Les produits PCR ont ensuite été clonés dans le vecteur pUNI/V5-His-TOPO, permettant l'obtention d'un vecteur .donneur (pUNI-*dsr*E) à recombiner avec un vecteur accepteur (pCR-T7-E) et adapté à l'expression dans *E. coli.* Le plasmide final a été désigné pCR-T7-*dsr*E.

Cette construction, plaçant le gène *dsr*E sous le contrôle du promoteur du bactériophage T7, a permis l'expression inductible du gène *dsr*E.

Après induction avec 1 mM d'IPTG, les cellules de *E. coli* BL21 transformées ont été récoltées par centrifugation après 4 heures de croissance, et re-suspendues à une densité optique finale de 80 à 600 nm dans du tampon acétate de sodium 20 mM pH 5,4 et du Triton X100 à 1% (v/v) en présence de 1 mM PMSF afin d'empêcher la protéolyse dans les extraits cellulaires après sonication.

Des expériences similaires, réalisée avec le système pBAD-TOPO Thiofusion, ont permis de construire le vecteur recombinant pBAD-TOPO-*dsr*E.

**Tests enzymatiques :**

Les réactions enzymatiques ont été réalisées dans les conditions standard à 30 °C dans du tampon acétate de sodium 20 mM pH 5,4, $NaN_3$ 1 $g/l^{-1}$ et saccharose 100 $g/l^{-1}$. L'activité de l'enzyme DSR-E a été déterminée en mesurant la vitesse de libération des sucres réducteurs, représentés en l'espèce par le fructose, à l'aide de la méthode à l'acide dinitro-salicylique bien connue de l'homme du métier. Une unité a été définie comme la quantité d'enzyme qui catalysait la formation d'1 μmol de fructose par minute dans les conditions standard. Les oligosaccharides ont été synthétisés dans un milieu réactionnel contenant 100 g/l de maltose, 200 g/l de saccharose et 0,5 unités/ml de DSR-E.

Comme pour la synthèse de dextrane, la réaction enzymatique a été poursuivie pendant 24 heures en présence de 100 g/l de glucose. Le dextrane produit a été précipité en présence d'éthanol 50 % (v/v) et lavé deux fois dans l'éthanol à 50 % (v/v) avant lyophilisation. Il a ensuite été dissous à 10 mg/ml dans du $D_2O$ et analysé par spectrométrie deRMrl du $^{13}C$.

**Séparation par HPLC** :

Des échantillons de 100 μl ont été prélevés et chauffés à 95 °C pendant 5 minutes, puis dilués dans de l'eau ultra-pure de manière à obtenir une concentration finale en sucres totaux inférieure à 5g.l$^{-1}$. Après centrifugation, les substrats résiduels et les différentes espèces formées ont été analysés par HPLC sur colonne C18 (Ultrasep 100, 6 μm, 5x300 mm, Bishoff Chromatography).

La séparation des oligosides a été réalisée à température ambiante, pendant 30 minutes, dans de l'eau ultra-pure appliquée à titre d'éluant à un débit de 0,5 ml/min. La détection a été accomplie par réfractométrie.

De telles conditions ont permis de séparer les espèces suivantes : fructose, maltose, leucrose, saccharose, ainsi que les oligosides dont le degré de polymérisation ne dépassait pas 6.

**Calcul des rendements** :

La méthode de calcul des rendements des réactions de synthèse d'oligosides a pris en compte la concentration résiduelle d'accepteur, conformément à la formule suivante :

R = {[GOS finaux] – [GOS initiaux]} / {0,474 x [saccharose consommé] + [accepteur consommé]}

où R représente le rendement réel de la réaction de synthèse des GOS totaux, les concentrations étant exprimées en g/l.

**Construction des différentes formes délétées de la protéine DSR-E** :

Les différentes formes délétées de la protéine DSR-E [Figure 1c) à 1i)] ont été obtenues par amplification par PCR des fragments correspondants du gène *dsr*E, puis clonage dans le vecteur pBAD-TOPO Thiofusion, dont la description est fournie supra. Les amorces utilisées pour l'amplification des régions sélectionnées à partir du gène *dsr*E sont listées dans le tableau II suivant. Les positions des amorces sont indiquées en référence à la séquence ID no. 5, relative à la séquence du gène *dsr*E. Les bases mutées afin d'introduire le site de restriction *Nco*I sont en caractères gras et le site *Nco*I résultant est souligné.

**TABLEAU II** :

| Désignation | Positions | Séquence 5'-3' |
|---|---|---|
| pBAD-PS/ZV-dir | 344-373 | G**CC**A**TG**GCAAATACGATTGCAGTTGACACG |
| pBAD-ZV/CD1-dir | 971-1001 | G**CC**AT**GG**ACGGTAAAACCTATTTTCTTGACG |
| pBAD-CD1/GBD-dir | 3656-3682 | TCCATGGGTGAAAAAACAAGCACCGGC |
| pBAD-GBD/CD2-dir | 6167-6189 | AC**CA**T**G**GATATGTCTACTAATGC |
| pBAD-CD1/GBD-inv | 3638-3658 | TAACTGTTTAGGCAAGAATCC |
| pBAD-GBD/CD2-inv | 6146-6172 | TAATGTATTAGTGAATAAGTATTCACC |
| pBAD-ent-inv | 8714-8737 | AATTTGAGGTAATGTTGATTTATC |

Les amorces directes et inverses ci-dessus ont été dessinées de manière à assurer la fusion traductionnelle de l'étiquette thioredoxine en N-terminal et l'étiquette polyhistidine en C-terminal des formes protéiques tronquées en respectant les cadres ouverts de lecture des régions codant lesdites formes.

Dans la mesure où le plasmide pBAD-TOPO Thiofusion contient un site de restriction spécifique de l'enzyme *Nco*I situé au niveau de l'extrémité 5' de la région codant la thioredoxine, un deuxième site *Nco*I a été introduit dans chaque amorce directe afin de pouvoir, le cas échéant, extraire ladite région.

Les réactions d'amplification par PCR ont été réalisées à l'aide du système « Expand Long Template » dans les conditions suivantes : une pré-dénaturation à 94 °C pendant 3 minutes, suivie de 25 cycles à 94 °C pendant 30 secondes, 52 °C pendant 30 secondes et 68 °C pendant.7 minutes.

Les produits d'amplification ainsi générés ont ensuite été clonés dans le vecteur pBAD-TOPO Thiofusion aux fins de la transformation subséquente de la souche *E. coli* TOP 10. Des clones recombinants ont été sélectionnés, leur profil de restriction analysé afin d'identifier, pour chacune des formes recherchées, un plasmide recombinant portant l'insertion orientée comme attendu.

***Exemple 1 : Caractérisation et purification de l'enzyme DSR-E* et *obtention du gène dsrE***

Les enzymes produites par les cultures de *L. mesenteroides* et obtenues sur gel de polyacrylamide en SDS tel que décrit dans la partie Matériels et Méthodes ont été isolées par découpe du gel.
Les GOS produits par les enzymes ainsi isolées ont été analysés par HPLC selon les méthodes décrites dans (1). L'enzyme dont l'activité était recherchée a été déduite de la nature des GOS produits. Après protéolyse trypsique et séparation par HPLC des peptides produits, 2 peptides : 29- FYFESGK et 24- FESQNNNP ont été séquencés et utilisés comme modèle pour la synthèse d'amorces nucléotidiques dégénérées.
Les différentes étapes d'amplification et de clonage sont représentées dans la figure 2. Le gène complet a été inséré dans le plasmide pCR-T7-E et exprimé dans *E. coli.*
La production d'une enzyme fonctionnelle est attestée par la production des GOS dont l'analyse HPLC est représentée dans la figure 6b).
On remarquera en particulier l'importance des pics 5 et 7 représentatifs des GOS à ramification $\alpha(1\rightarrow2)$.

***Exemple 2 : caractérisation des séquences de dsrE et DSR-E***

2.1 Séquence nucléotidique :

La séquence nucléotidique de l'enzyme est représentée dans la séquence ID No. 4. Elle est composée d'un cadre de lecture de 8506 nucléotides.
La séquence nucléotidique de l'insert dans le plasmide pCR-T7-*dsr*E contient un site de liaison au ribosome (RBS), 9 bases en amont du codon d'initiation ATG et est composée d'un hexa-nucléotide GAGGAA.

2.2 Analyse de la séquence d'amino-acides :

La séquence de 8506 nucléotides de *dsr*E code une protéine de 2835 acides aminés représentée dans la séquence ID No. 2. Le point isolectrique de cette protéine est de 4,88 et son poids moléculaire théorique de 313,2 kDa. En dépit des fortes similarités avec les dextrane-saccharases déjà connues, DSR-E est caractérisée par une structure originale.
L'alignement de la séquence d'amino-acides avec l'ensemble des glycosyltransférases et des dextrane-saccharases connues confirme que la structure en domaine des glycosyltransférases et des dextrane-saccharases est conservée, à savoir : une séquence signal, une région variable, un domaine catalytique hautement conservé et un domaine de liaison au glucane. Cette structure est représentée dans la figure 1a).
Comme l'indique la figure 1b), un deuxième domaine catalytique forme la partie carboxy-terminale de l'enzyme comme cela été confirmé par PRODOM et une analyse Blast.
Avec un poids moléculaire de 313,2 kDa, DSR-E a environ 2 fois le poids moléculaire moyen des autres glycosyltransférases et dextrane-saccharases (1), ce qui est en accord avec la présence d'un deuxième domaine catalytique à l'extrémité C-terminale et également avec un domaine de liaison au glucane plus long.

a) analyse de la séquence signal :

La séquence signal et la séquence nucléotidique codant le peptide signal sont extrêmement conservées si on les compare aux autres dextrane-saccharases comme ceci est indiqué dans la figure 3. Le site de clivage est localisé entre les acides aminés 40 et 41.

b) domaine variable :

En aval du peptide signal, DSR-E a un domaine variable de 207 acides aminés. Lorsqu'on le compare aux autres domaines variables des glycosyltransférases, en utilisant un programme d'alignement de type SAPS, on met en évidence la présence d'un motif de 14 acides aminés répété 11 fois comme ceci est indiqué dans la figure 4.
Ce motif répété, riche en alanine, threonine et acide aspartique n'a jamais été identifié précédemment.
Le rôle et la signification de cette région n'ont jamais été élucidés. Différentes études ont démontré que sa délétion n'affecte pas l'activité enzymatique (4). Le rôle du motif répété de 14 acides aminés qui n'existe pas dans les autres glycosyltransférases reste néanmoins à déterminer.

c) analyse des domaines catalytiques :

Le premier domaine catalytique s'étend des acides aminés 248 à 1142 (CD1) de la séquence ID No. 2, alors que le deuxième est localisé entre les acides aminés 1980 et 2836 (CD2). Ces deux domaines présentent 45 % d'identité et 65 % de similarité entre eux.

CD1 et CD2 contiennent les acides aminés déjà identifiés dans les glycosyltransférases et les dextrane-saccharases comme étant essentiels à leur activité enzymatique, et comme ceci est indiqué dans la figure 5.

Les triades catalytiques de CD1 et CD2 déterminées par analogie avec l'$\alpha$ amylase (7) sont présentes aux positions suivantes : (Asp 527/Glu 565/Asp 638 pour CD1 et Asp 2210/Glu 2248/Asp 2322 pour CD2).

D'autres résidus conservés ont été identifiés comme importants pour l'activité enzymatique : les résidus Trp 425/Glu 430 pour CD1 et Trp 2122/Glu 2127 pour CD2, lesquels sont analogues à celles du domaine N-terminal de GFTI décrits par Monchois *et al.* (4) : Trp 344/Glu 349.

En revanche, certaines séquences situées dans la région conservée des glycosyltransférases et des dextrane-saccharases ne se retrouvent pas dans CD2 de DSR-E. Ainsi, comme indiqué dans la figure 5 ci-dessous, les séquences FIHNDTI (2214-2220) et KGVQEKV (2323-2329) divergent des autres séquences consensus des dextrane-saccharases déjà étudiées qui sont respectivement NVDADLL et SEVQTVI.

d) domaine de liaison au glucane :

Lorsque l'on compare la séquence de DSR-E avec les séquences connues, il apparaît que le domaine de liaison au glucane est sensiblement plus long. En effet, ce domaine a une longueur d'environ 500 acides aminés dans les glycosyltransférases et les dextrane-saccharases étudiées alors que dans DSR-E, il représente 836 acides aminés. Plusieurs motifs répétés A et C et, en particulier, une série de répétitions AC ont pu être identifiés. Dans le Tableau III suivant, sont indiquées les séquences consensus des motifs répétés du GBD, notamment des motifs A et C, décrits dans la littérature relativement à des dextrane-saccharases de *Leuconostoc* et *Streptococcus* spp.

**TABLEAU III** :

| Motif | Séquence consensus |
|---|---|
| A | WWYFNxDGQAATGLQTIDGQTVFDDNGxQVKG |
| B | VNGKTYYFGSDGTAQTQANPKGQTFKDGSGVLRFYNLEGQYVSGSGWY |
| C | DGKIYFFDPDSGEVVKNRFV |
| D | GGWKNADGTYSKY |
| N | YYFxAxQGxxxL |
| x : acide aminé indifférent. | |

***Exemple 3 : expression de dsrE chez E. coli***

Des cellules de *E. coli* BL21 (DE3) pLysS pCR-T7-*dsr*E ont été cultivées comme décrit ci-dessus. Après gel d'électrophorèse en polyacrylamide-(page-SDS) l'analyse des extraits protéiques a révélé effectivement la présence de plusieurs bandes ayant l'activité de dextrane saccharase, ladite activité étant mesurée comme ci-dessus.

La lignée *E. coli* JM109 [pCR-T7-*dsr*D] a été déposée à la CNCM le 15 mars 2001 sous le numéro I-2649.

Identification et caractérisation de l'activité enzymatique :

En utilisant une molécule accepteur de glucose, les dextrane-saccharases produites par *E. coli* recombinant ont été comparées avec celles produites par *L. mesenteroides* NRRL B-1299.

L'analyse HPLC des produits de la réaction avec la DSR-E recombinante montre (figure 6) des temps de rétention correspondant aux GOS préalablement identifiés R4 et R5 (2). Les oligosaccharides de type R sont les séries de GOS linéaires, le lien $\alpha(1\rightarrow2)$ étant lié à l'extrémité non-réductrice. La série OD, GOS linéaires résultant de liens glycosidiques $\alpha(1\rightarrow6)$ avec un résidu maltose à l'extrémité réductrice, a été observée en très faibles quantités. Trois nouveaux composés sont en revanche détectés dans les produits de l'enzyme recombinante.

Identification des GOS produits :

Finalement, la figure 6b montre clairement que les pics 5 et 7 représentant les GOS de la série R sont relativement plus importants avec l'enzyme recombinante qu'avec l'enzyme native dont les pics correspondant au panose et à OD5 sont plus importants.

***Exemple 4 : Effet de la délétion de CD2 sur l'activité enzymatique de DSR-E***

L'ADN génomique de *L. mesenteroides* NRRL B-1299 a été utilisé comme matrice pour amplifier par PCR le gène *dsr*E délété de la séquence correspondant au second domaine catalytique. Pour cela, 2 oligonucléotides, ECHO-dir (5'-AGTTGTATGAGAGACATGAGGGTAATTTGTGACCGTAAAAAATTG) (SEQ. ID No. 6), correspondant à la séquence nucléotidique -6 à 39 et contenant le codon d'initiation de la traduction, et ECHO-inv-dél (5'-GTATTAGTGAATAAGTATTCACCATTGCATTTATCGTCAAAATAGTACG) (SEQ. ID No. 7) complémentaire de la séquence 5889-5937 et correspondant à la séquence peptidique YYFDDKGNGEYCFTNT, ont été synthétisés, afin de fusionner l'extrémité C-terminale de la protéine délétée avec un tag His présent sur le vecteur de clonage. La réaction PCR a été réalisée grâce à un DNA thermal cycler model 2400 (Perkin-Elmer), avec le système Expand Long Template System (Boehringer Mannheim), suivant le cycle de température : 94°C pendant 3 min, puis 25 cycles avec: 30 s à 94°C, 30s à 55°C et 7 min à 68°C. Le produit PCR a ensuite été cloné dans le vecteur donneur pUNI, et le plasmide résultant, utilisé dans une réaction de recombinaison avec le vecteur d'expression pCR-T7-Δ*dsr*E.

La préparation des extraits cellulaires, les réactions enzymatiques, l'analyse des produits de la réaction sont les mêmes que dans l'exemple 3 ci-dessus.

Le profil HPLC des GOS obtenus avec l'enzyme DSR-E délétée du domaine CD2 apparaît dans la figure 6 c).

Les GOS de type R, représentés par les pics 5 et 7 visibles dans la figure 6 a) et 6 b) sont totalement absents des produits obtenus avec l'enzyme recombinante délétée de CD2. Les seuls produits analysables sont ceux correspondant à des oligosides linéaires résultant de liens $\alpha(1 \rightarrow 6)$ avec un résidu maltose dans la partie réductrice. Ce résultat indique clairement le rôle essentiel du domaine catalytique situé dans la partie carboxy-terminale de l'enzyme dans sa capacité à former des liaisons osidiques $\alpha(1 \rightarrow 2)$.

***Exemple 5 : étude des relations structure-fonction de la protéine DSR-E***

Le gène *dsr*E, en ce qu'il est le premier gène codant une dextrane-saccharase catalysant la synthèse de liaisons $\alpha(1\rightarrow2)$ à avoir été cloné, revêt un intérêt particulier. Il est donc important de caractériser ce gène ainsi que son produit d'expression, en l'occurrence en déterminant quelle est l'implication des différents domaines composant la protéine DSR-E dans la fonction qui lui a été assignée, à savoir de correspondre à une dextrane-saccharase spécifique de la synthèse de liaisons $\alpha(1\rightarrow2)$.

5.1 <u>Formes délétées de la protéine DSR-E</u> :

L'étude de six formes différentes obtenues par délétion d'un ou de plusieurs domaines de la protéine DSR-E a été envisagée afin de déterminer, en référence à la Figure 1 ci-dessous : (i) l'influence de la présence du domaine CD2 en étudiant les constructions GBD-CD2 et Δ(CD2) ; (ii) l'influence de la présence de la zone variable en analysant les formes Δ(ZV) et CD1-GBD ; et (iii) le potentiel catalytique intrinsèque des domaines CD1 et CD2 exprimés de façon isolée (constructions CD1 et CD2).

L'activité catalytique de chacune des différentes formes a été comparée à celle observée avec le contrôle correspondant à la forme entière délétée du seul peptide signal Δ(PS) [Figure 1c)].

5.2 <u>Analyse des constructions</u> :

A l'issue de la procédure expérimentale d'amplification par PCR et de clonage détaillée supra, plusieurs clones présentant une insertion selon l'orientation escomptée ont été obtenus pour chacune des constructions envisagées, à l'exception de la forme tronquée GBD-CD2 pour laquelle le produit d'amplification souhaité n'a pu être cloné.

Les séquences des insertions ont été déterminées afin de s'assurer de l'absence de mutations susceptibles de modifier, après traduction, des acides aminés situés au niveau de positions présumées essentielles pour l'activité enzymatique de la protéine ainsi codée.

Une mutation a été identifiée au niveau de la $31^{ème}$ base de l'insertion relative au contrôle Δ(PS), induisant la substitution d'un acide aspartique par une asparagine en position 10 de la zone variable. N'étant pas située au niveau des motifs répétés S de la zone variable (Figure 4), il semblerait que l'incidence de cette mutation sur la fonction finalement observée soit négligeable.

Une mutation a été introduite dans le produit d'amplification correspondant à la construction Δ(CD2), modifiant le résidu aromatique F1411 en leucine. Cette mutation est située dans le premier tiers du domaine de liaison au glucane GBD, au niveau d'une jonction entre deux motifs répétés.

La construction Δ(ZV), eu égard aux erreurs effectuées par la polymérase lors de la réaction d'amplification par PCR, ne présentait pas la séquence attendue. En effet, l'insertion contenait un cadre ouvert de lecture, ledit cadre correspondant pour l'essentiel à la forme GBD-CD2 qui n'avait pu être clonée. Cependant, dans la forme GBD-CD2 obtenue en définitive à la place de Δ(ZV), les 46 résidus N-terminaux étaient absents. Or, le domaine GBD compte plus de 800 acides aminés formant un enchaînement de 24 unités répétées. Cet enchaînement est tel que, sur les 46 résidus tronqués, seuls les 9 derniers étaient situés au niveau de l'une desdites unités, et en particulier au niveau de la première d'entre elles. Il paraissait en conséquence plausible de considérer que la délétion de ces acides aminés était sans influence sur la réaction enzymatique catalysée par la forme protéique correspondante. Cette hypothèse était d'autant plus vraisemblable qu'il a été montré sur d'autres dextrane-saccharases, que la perte d'un certain nombre d'unités répétées du domaine GBD ne réduisait pas l'activité de la protéine résultante de manière significative (8).

L'insertion codant la forme CD1-GBD contenait une mutation affectant le résidu F633, situé dans le domaine CD1, et plus précisément, au niveau d'une région fortement conservée parmi les dextrane-saccharases, elle-même localisée juste avant le deuxième acide aspartique de la triade catalytique (Figure 5). La phénylalanine attendue était substituée par une leucine. Il est difficile, à ce stade, d'estimer l'impact d'une telle mutation sur l'activité catalytique observée.

De la même manière que pour les autres constructions, la séquence des insertions codant les domaines catalytiques CD1 et CD2 isolés est déterminée.

5.3 Produits d'expression et activités enzymatiques :

Les protéines correspondant aux diverses formes délétées de DSR-E ont été exprimées en soumettant les cellules recombinantes de E. *coli* à une induction par le L-arabinose selon une concentration de 0,002 %. L'activité enzymatique a été observée pendant les quatre premières heures qui suivaient l'induction.

Les extraits protéiques obtenus par sonication des culots cellulaires ont été analysés en électrophorèse SDS-PAGE (Sambrook et Russel, 2001, supra). Les masses moléculaires des protéines recombinantes ont été estimées à partir des profils électrophorétiques obtenus, lesdites masses correspondant pour l'essentiel aux masses attendues, en tenant compte de l'incrémentation de 12 kDa liée à l'étiquette thioredoxine. Le Tableau IV ci-après résume les valeurs estimées des masses moléculaires des différentes formes tronquées et fournit, à titre de comparaison, les masses attendues.

**TABLEAU IV** :

| Forme protéique | Masse attendue (kDa) | Masse attendue + thioredoxine (kDa) | Masse estimée (kDa) |
|---|---|---|---|
| Δ(PS) | 309 | 321 | 324 |
| Δ(CD2) | 218 | 230 | ND |
| GBD-CD2 | 224 | / | 233 |
| CD1-GBD | 193 | 205 | 199 |
| CD1 | 99 | 111 | 111 |
| CD2 | 95 | 107 | ND |

ND : non déterminé.

Dans le Tableau V suivant, sont indiquées la nature et la position des acides aminés marquant le début et la fin des formes protéiques construites dans le cadre de cette étude. Les différentes positions font référence à la séquence ID no. 2 correspondant à la protéine DSR-E.

**TABLEAU V** :

| Forme protéique | Acide aminé de début | Acide aminé de fin | Longueur totale |
|---|---|---|---|
| Δ(PS) | N41 | I2835 | 2795 |
| Δ(CD2) | M1 | L1980 | 1980 |
| GBD-CD2 | M1188 | I2835 | 1648 |
| CD1-GBD | I248 | L1980 | 1733 |
| CD1 | I248 | Q1141 | 894 |
| CD2 | D1981 | I2835 | 855 |

La forme GBD-CD2 ne possédait pas l'étiquette thioredoxine. En effet, cette forme était issue des aléas expérimentaux occasionnés par la procédure d'amplification par PCR de la séquence censée coder la forme Δ(ZV). Eu égard aux délétions de séquences alors générées, l'étiquette thioredoxine, en principe située en 5' de la protéine d'intérêt, n'avait pu être fusionnée avec la région GBD-CD2.

La qualité des gels d'électrophorèse n'a pas permis de déterminer si le niveau d'expression des différentes formes était quantitativement identique et, en conséquence, si lesdites formes étaient présentes dans les mêmes proportions dans les extraits cellulaires.

Aussi les mesures d'activité fournies ont été établies sur la base d'un volume donné d'extraits cellulaires, mais n'ont pu être ramenées à la quantité de chaque protéine réellement contenue dans ledit volume d'extraits.

La synthèse de polymères de dextrane *in situ* par incubation des gels d'électrophorèse dans une solution de saccharose, et la coloration au réactif de Schiff subséquente ont confirmé la présence de protéines possédant une activité glucane-saccharase dans les extraits cellulaires correspondant à Δ(PS), Δ(CD2), GBD-CD2 et CD1-GBD.

Dans le Tableau VI ci-dessous, sont présentées les activités enzymatiques maximales observées pour chaque construction. Les résultats ont confirmé les données tirées des expériences de coloration des gels au réactif de Schiff, à savoir le fait que les extraits cellulaires relatifs aux formes Δ(PS), Δ(CD2), GBD-CD2 et CD1-GBD présentaient une activité saccharase, à l'inverse des deux domaines catalytiques pris isolément. Ce résultat était conforme à la littérature, étant donné qu'il avait été montré pour d'autres dextrane-saccharases, que l'absence du domaine GBD induisait une perte d'activité enzymatique drastique (8, 9, 10).

**TABLEAU VI :**

| Forme protéique | Δ(PS) | Δ(CD2) | GBD-CD2 | CD1-GBD | CD1 | CD2 |
|---|---|---|---|---|---|---|
| Activité maximale (U/l) | 1063 | 181 | 86 | 235 | 5,3 | 0 |

La forme CD1 possédait une activité intrinsèque de niveau suffisant pour être détectée. Quant à la forme GBD-CD2, elle présentait un niveau d'activité non négligeable, dont on pouvait conclure que l'organisation structurale correspondante, à savoir un domaine catalytique en aval du domaine de liaison au glucane, demeurait enzymatiquement active.

5.4 <u>Effets des délétions sur la synthèse d'oligosides</u> :

Dans la mesure où la spécificité de synthèse des liaisons α(1→2) est conservée lors de la réaction en présence d'un accepteur, des expériences de synthèse d'oligosides à partir de maltose ont d'abord été effectuées (Figure 7).

Lorsque les réactions ont été menées à leur terme, c'est-à-dire lorsque tout le saccharose a été consommé, les rendements de synthèse en oligosides ont été calculés. Les résultats apparaissent dans le Tableau VII suivant. Seule la réaction impliquant l'extrait cellulaire contenant la forme protéique CD1 n'a pu permettre un tel calcul. L'effet de la température a probablement conduit à l'inactivation de la très faible activité présente dans l'extrait protéique.

**TABLEAU VII** :

| Forme protéique | Rendement en oligosides de la série OD (%) | Rendement en oligosides de la série R (%) | Rendement en oligosides totaux |
|---|---|---|---|
| Enzyme native | 36 | 28 | 64 |
| Δ(PS) | 41 | 14 | 55 |
| ΔCD2) | 67 | 1 | 68 |
| GBD-CD2 | 45 | 47 | 92 |
| CD1-GBD | 100 | 0 | 100 |

Comme indiqué sur la Figure 7 ci-dessous, la présence d'oligosides de la série R n'était décelée qu'avec les formes enzymatiques possédant le domaine catalytique CD2, à l'exception du cas où ledit domaine était isolé et alors rendu complètement inactif. En effet, les temps de rétention des oligosides synthétisés par la forme délétée du deuxième domaine catalytique, ainsi que par la forme CD1-GBD, correspondaient uniquement à ceux de la série OD, c'est à dire aux GOS dépourvus de liaisons α(1→2). Ces résultats indiquaient donc que le domaine CD2 était requis pour la formation des liaisons α(1→2).

Les produits obtenus avec la forme GBD-CD2 ont permis d'étayer ces observations. Cette construction, qui possédait CD2 comme seul domaine catalytique, était capable de catalyser de manière prépondérante la synthèse d'oligosides de la série R, possédant des liaisons α(1→2). Ce résultat démontrait donc que la spécificité en terme de fonction de l'enzyme DSR-E réside dans la séquence très originale de ce domaine, et non pas dans l'association de deux domaines catalytiques. En outre, la forme protéique GBD-CD2 permettait également la synthèse de liaisons α(1→6). Toutefois, les faibles rendements obtenus pour ces oligosides indiquaient qu'ils étaient préférentiellement convertis en oligosides de degré de polymérisation supérieur appartenant à la série R, ce qui empêchait leur accumulation dans le milieu réactionnel, à la différence des molécules de la série R qui, elles, n'étaient pas converties (2).

En comparant les profils des produits obtenus, tels que montrés à la Figure 7, il est apparu que la forme entière Δ(PS) synthétisait majoritairement des oligosides linéaires. En effet, la molécule R4 était absente et l'oligoside R5 faiblement présent. Le domaine catalytique CD1 permettait, quant à lui, de catalyser la synthèse exclusive de liaisons α(1→6) et son activité semblait prépondérante par rapport à celle du domaine CD2. Aussi, dans la forme entière de l'enzyme, l'implication du domaine CD2 serait donc moins importante du fait de : (i) paramètres catalytiques intrinsèques plus faibles ; et/ou (ii) d'une configuration globale de l'enzyme défavorable à son activité.

En outre, l'enzyme entière Δ(PS) catalysait la synthèse d'oligosides de la série R avec un rendement inférieur à celui observé avec le mélange de dextrane-saccharases produit par *L. mesenteroides* NRRL B-1299 (Figure 7). Le rendement obtenu, de 28 %, était situé entre ceux observés avec la forme entière Δ(PS) d'une part, et la forme GBD-CD2 d'autre part. Or, l'on sait que la souche sauvage produit plusieurs formes de dextrane-saccharases susceptibles de synthétiser des liaisons osidiques, et notamment des liaisons α(1→2). Une hypothèse avait été émise, selon laquelle lesdites formes étaient des produits de dégradation de DSR-E. Ainsi, dans la mesure où des formes tronquées de DSR-E, telles GBD-CD2, pouvaient catalyser la synthèse d'oligosides de la série R plus efficacement, il semblerait que les rendements obtenus avec le mélange hétérogène produit par *L. mesenteroides* NRRL B-1299 soient imputables à la contribution des activités catalytiques de l'ensemble de ces différentes formes enzymatiques.

En conclusion, les inventeurs ont réussi, en isolant une dextrane saccharase particulière produite par L. mesenteroides, à caractériser une structure particulière et inattendue de cette enzyme apte à produire des oligosides d'intérêt et présentant des ramifications de type α(1→2). L'identification et la caractérisation de cette séquence permettent d'une part de construire des cellules ou organismes recombinants exprimant de façon spécifique cette enzyme et, d'autre part, d'en envisager sa modification par mutagenèse dirigée ou aléatoire ou par évolution moléculaire (DNA Shuffling) afin d'en améliorer encore ses caractéristiques.

Cet apport permet en outre d'améliorer le rendement et la reproductibilité de la production des GOS d'intérêt pour les différentes applications citées ci-avant.

**REFERENCES BIBLIOGRAPHIQUES**

(1) Monchois V., Willemot R.M., Monsan P. (1999). Glucansucrases : mechanism of action and structure-function relation-ships. FEMS microbiol. Rev. 23, 131-151.

(2) Dols M., Remaud-Simeon M., Willemot R.M., Vignon M.R., Monsan P.F. (1998). Structural characterization of the maltose acceptor-products synthesised by Leuconostoc mesenteroides NRRL B-1299 dextransucrase. Carbohydrate Research 305, 549-559.

(3) Arnold F.H. (2001). Nature 409 n° 6817, 253.

(4) Monchois V. Vignon M., Russel R.R.B. (1999). Isolation of key amino-acid residues at the N-terminal end of the core region of Streptococcus downei glucansucrase GTF-I. Appl. Microbiol. Biotechnol. 52, 660-665.

(5) Wilke-Douglas M., Perchorowicz J.T., Houck C.M., Thomas B.R. (1989). Methods and compositions for altering physical characteristics of fruit and fruit products. PCT patent, WO 89/12386.

(6) Arguello-MOrales M.A., Remaud-Simeon M., Pizzut S., Sarçabal P., Willemot R.M., Monsan P. (2000). Sequence analysis of the gene encoding alternansucrase, a sucrose glucosyltransferase from Leuconostoc mesenteroides NRRL B-1355. FEMS Microb. Lett. 182, 81-85.

(7) Devulapalle K.S., Goodman S., Gao Q., Hemsley A., Mooser G. (1997). Knowledge-based model of a glusocyltransferase from oral bacterial group of mutants streptococci. Protein Sci. 6, 2489-2493.

(8) Kato C., et Kuramitsu H.K. (1990). Carboxy-terminal deletion analysis of the Streptococcus mutans glucosyltransferase-I enzyme. FEMS Microbiol. Lett. 72, 299-302.

(9) Lis M., Shiroza T., et Kuramitsu H.K. (1995). Role of the C-terminal direct repeating units of the Streptococcus mutans glucosyltransferase-S in glucan binding. Appl. Env. Microbiol. 61, 2040-2042.

(10) Monchois V., Remaud-Simeon M., Russel R.R.B., Monsan P., et Willemot R.M. (1997). Characterization of Leuconostoc mesenteroides NRRL B-512F dextransucrase (DSR-S) and identification of amino-acid residues playing a key role in enzyme activity. Appl. Microbiol. Biotechnol. 48, 465-472

## Revendications

1. Polypeptide isolé ayant une activité enzymatique de glycosyltransférase apte à former des dextranes présentant des ramifications $\alpha(1\rightarrow2)$ à partir de saccharose, d'α-D-fluoro-glucose, de para-nitrophényl-α-D-glucopyranoside, d'-α-D-glucopyranoside-α-D-sorbofurano-side ou de 4-O-α-D-galactopyranosylsucrose, ledit polypeptide étant **caractérisé** :

   a. **En ce qu'**il comprend la SEQ.ID NO : 2, ou
   b. **En ce qu'**il a une structure constituée, partant de la partie aminée du polypeptide, d'une séquence signal, d'une région variable, d'un premier domaine catalytique, d'un domaine de liaison au glucane et d'un deuxième domaine catalytique, dans le cadre de laquelle :

      i. les séquences des premier et deuxième domaines catalytiques dudit polypeptide ont un pourcentage de similarité avec la SEQ. ID NO : 1 compris entre 65 % et 100 %, la triade catalytique Asp/Glu/Asp en positions respectives 230/268/342 de la SEQ ID NO : 1 étant conservée, et
      ii. le domaine de liaison au glucane a une la taille supérieure à 500 acides aminés.

2. Polypeptide selon la revendication 1, dans lequel les acides aminés suivants sont inchangés par rapport à la séquence de SEQ ID NO : 2 :

   W en positions 425 et 2122,
   E en positions 430, 565 et 2127, 2248,
   D en positions 487, 489, 527, 638, 2170, 2172, 2210 et 2322,
   H en position 637 et 2321,
   Q en position 1019 et 2694.

**3.** Acide nucléique isolé codant une enzyme à activité glycosyltransférase apte à former des dextranes présentant des ramifications α(1 → 2) à partir de saccharose, d'α-D-fluoro-glucose, de para-nitrophényl-α-D-glucopyranoside, d'aD-glucopyranoside-αD-sorbofurano-side ou de 4-O-α-D-galactopyranosylsucrose, ledit acide nucléique étant **caractérisé** :

a) **En ce qu'**il comprend ou consiste en la SEQ. ID NO : 4 ; ou
b) **En ce qu'**il comprend, de la partie 5' à la partie 3', une séquence codant un peptide signal, une séquence variable, une séquence codant un premier domaine catalytique, une séquence codant un domaine de liaison au glucane et une séquence codant un deuxième domaine catalytique, et :

i. les deux séquences d'acide nucléique codant les domaines catalytiques ont au moins 80 % d'identité avec la séquence SEQ ID NO : 3, le codage de la triade catalytique Asp/Glu/Asp codée par les triplets GAT, GAA et GAT trouvés respectivement en positions 6628/6742/6964 de la SEQ ID NO : 4 étant conservé, et
ii. la séquence d'acide nucléique codant un domaine de liaison au glucane code un domaine de liaison au glucane qui a une la taille supérieure à 500 acides aminés.

**4.** Acide nucléique isolé selon la revendication 3, comprenant une séquence ayant au moins 80 % d'identité avec la séquence codant une dextrane saccharase exprimée par le plasmide pCR-T7-*dsr*D déposé à la CNCM le 15 mars 2001 sous le numéro I-2649, dans laquelle le codage de la triade catalytique Asp/Glu/Asp codée par les triplets GAT, GAA et GAT trouvés respectivement en positions 6628/6742/6964 de la SEQ ID NO : 4 est conservé.

**5.** Vecteur d'expression comprenant un acide nucléique selon l'une quelconque des revendications 3 ou 4.

**6.** Vecteur selon la revendication 5, dans lequel l'acide nucléique est sous le contrôle de séquence permettant son expression dans des cellules procaryotes ou eucaryotes.

**7.** Cellule hôte comprenant un acide nucléique selon la revendication 3 ou 4 ou un vecteur selon l'une des revendications 5 ou 6 choisie dans un groupe comprenant *E. coli*, les végétaux, les *Lactococcus* les *Bacillus* et les levures.

**8.** Cellule selon la revendication 7, **caractérisée en ce qu'**il s'agit d'une souche de *E. coli* déposée à la CNCM le 15 mars 2001 sous le n° I-2649.

**9.** Procédé de production d'une dextrane saccharase apte à former des liaisons α1 → 2 comprenant :

a) l'insertion d'un acide nucléique selon l'une des revendications 3 ou 4 ou un vecteur selon l'une des revendications 5 ou 6 dans une cellule hôte selon la revendication 7;
b) la purification de l'enzyme à partir d'un extrait cellulaire.

**10.** Procédé selon la revendication 9, dans lequel la cellule hôte est un eucaryote choisi dans un groupe comprenant les levures, les champignons, et les végétaux.

**11.** Procédé d'obtention d'une glycosyltransférase isolée apte à former des oligosides ou des dextranes présentant un taux de liaisons α(1 → 2) supérieur à 30 % comprenant :

- une étape de modification aléatoire de la séquence SEQ ID NO :4 et d'établissement d'une banque de variants,
- une étape d'expression de ces séquences modifiées dans une cellule hôte appropriée, un hôte abritant un variant,
- une étape de criblage des hôtes exprimant une enzyme apte à former plus de 30 % de liaisons α(1 → 2) sur un substrat approprié,
- une étape d'isolement du ou des gènes améliorés.

**12.** Utilisation d'une glycosyltransférase obtenue par un procédé selon l'une des revendications 9 ou 10 dans la fabrication d'une composition pharmaceutique ou cosmétique.

## Patentansprüche

1. Isoliertes Polypeptid mit einer Glycosyltransferase-Enzymaktivität, die geeignet ist, Dextrane zu bilden, die Verzweigungen α(1->2) ausgehend, von Saccharose, α-D-Fluor-Glukose, Para-Nitrophenyl-α-D-Glucopyranoside, α-D-Glucopyranosid-α-D-Sorbofuran-Sid oder 4-O-α-D-Galactopyranosylsukrose aufweisen, wobei das genannte Polypeptid **dadurch gekennzeichnet ist**:

   a. **dass** es die SEQ ID NR. 2 umfasst oder
   b. **dass** es eine Struktur hat, die ausgehend von dem Amino-Teil des Polypeptids aus einer Signalsequenz, einer variablen Region, einem ersten katalytischen Bereich, einer Verbindungsdomäne mit Glucan und einem zweiten katalytischen Bereich gebildet ist, in deren Rahmen:

      i. die Sequenz der ersten und zweiten katalytischen Bereiche des genannten Polypeptids einen Ähnlichkeitsprozentsatz mit der SEQ ID NR. 1 haben, der zwischen 65 % und 100 % inbegriffen ist, wobei die katalytische Triade Asp/Glu/Asp in den jeweiligen Positionen 230/268/342 der SEQ ID NR. 1 beibehalten ist, und
      ii. der Verbindungsbereich mit dem Glucan eine größere Größe als 500 Aminosäuren hat.

2. Polypeptid gemäß Anspruch 1, bei dem die folgenden Aminosäuren im Verhältnis zur Sequenz der SEQ ID NR. 2 unverändert sind:

   W in den Positionen 425 und 2122,
   E in den Positionen 430, 565 und 2127, 2248,
   D in den Positionen 487, 489, 527, 638, 2170, 2172, 2210 und 2322,
   H in den Positionen 637 und 2321,
   Q in den Positionen 1019 und 2694.

3. Isolierte Nukleinsäure, die für ein Enzym mit Glycotransferase-Aktivität kodiert, das geeignet ist, Dextrane zu bilden, die Verzweigungen α(1 -> 2) ausgehend von Saccharose, α-D-Fluor-Glukose, Para-Nitrophenyl-α-D-Glucopyranosid, α-D-Glucopyranosid-α-D-Sorbofuran-Sid oder 4-O-α-D-Galactropyranosylsukrose aufweisen , wobei die genannte Nukleinsäure **dadurch gekennzeichnet ist**:

   a) **dass** sie die SEQ ID NR. 4 umfasst oder daraus besteht; oder
   b) **dass** sie vom Teil 5' bis zum Teil 3' eine für ein Signalpeptid kodierende Sequenz, eine variable Sequenz, eine einen ersten katalytischen Bereich kodierende Sequenz, eine eine Verbindungsdomäne mit dem Glucan kodierende Sequenz und eine eine zweite katalytische Domäne kodierende Sequenz umfasst und:

      i. die zwei Nukleinsäuresequenzen, die für die katalytischen Bereiche kodieren, eine wenigstens 80 %i-ge Identität mit der Sequenz SEQ ID NR. 3 aufweisen, wobei die Kodierung der katalytischen Triade Asp/Glu/Asp, die von den Triplets GAT, GAA und GAT kodiert ist, die jeweils in den Positionen 6628/6742/6964 des SEQ ID NR. 4 angeordnet sind, beibehalten ist, und
      ii. wobei die Nukleinsäuresequenz, die für eine Verbindungsdomäne mit dem Glucan kodiert, eine Verbindungsdomäne mit dem Glucan kodiert, die eine größere Größe hat als 500 Aminosäuren.

4. Isolierte Nukleinsäure gemäß Anspruch 3, umfassend eine Sequenz mit wenigstens 80 %iger Identität mit der Sequenz, die für eine Dextransaccharase kodiert, die von dem Plasmid pCR-T7-*dsr*D exprimiert ist, das am 15. März 2001 bei der CNCM unter der Nummer I-2649 angemeldet wurde, in der die Kodierung der katalytischen Triade Asp/Glu/Asp, die von den Triplets GAT, GAA und GAT kodiert ist, die jeweils in den Positionen 6628/6742/6964 des SEQ ID NR. 4 angeordnet sind, beibehalten ist.

5. Expressionsvektor, umfassend eine Nukleinsäure gemäß irgendeinem der Ansprüche 3 oder 4.

6. Vektor gemäß Anspruch 5, bei dem die Nukleinsäure unter der Sequenzkontrolle steht, die ihre Expression in prokaryotischen oder eukaryotischen Zellen zulässt.

7. Wirtszelle, umfassend eine Nukleinsäure gemäß Anspruch 3 oder 4 oder einen Vektor gemäß einem der Ansprüche 5 oder 6, die aus einer Gruppe ausgewählt ist, umfassend *E. coli*, Gewächse, *Lactococcus, Bacillus* und Hefen.

8. Zelle gemäß Anspruch 7, **dadurch gekennzeichnet, dass** es sich um einen Stamm von *E. coli* handelt, der am 15. März 2001 unter der Nr. I-2649 bei der CNCM angemeldet wurde.

9. Produktionsverfahren einer Dextransaccharase, die geeignet ist, Verbindungen $\alpha$1 -> 2 zu bilden, umfassend:

a) das Einfügen einer Nukleinsäure gemäß einem der Ansprüche 3 oder 4 oder eines Vektors gemäß irgendeinem der Ansprüche 5 oder 6 in eine Wirtszelle gemäß Anspruch 7;
b) die Reinigung des Enzyms ausgehend von einem Zellextrakt.

10. Verfahren gemäß Anspruch 9, bei dem die Wirtszelle ein Eukaryot ist, das aus einer Gruppe ausgewählt ist, umfassend Hefen, Pilze und Gewächse.

11. Verfahren zum Erhalt einer isolierten Glycosyltransferase, die geeignet ist, Oligoside oder Dextrane zu bilden, die einen Verbindungsgrad $\alpha$(1 -> 2) von größer als 30 % aufweist, umfassend:

einen zufälligen Modifikationsschritt der Sequenz SEQ ID NR. 4 und der Herstellung einer Bank von Varianten,
- einen Expressionsschritt dieser modifizierten Sequenzen in einer geeigneten Wirtszelle, einen eine Variante beherbergenden Wirt,
- einen Durchsiebschritt der Wirte, die ein Enzym exprimieren, das geeignet ist, mehr als 30 % Verbindungen $\alpha$(1 -> 2) auf einem geeigneten Substrat zu bilden,
- einen Isolationsschritt des oder der verbesserten Gens / Gene.

12. Verwendung einer Glycosyltransferase, die durch ein Verfahren gemäß einem der Ansprüche 9 oder 10 bei der Herstellung einer pharmazeutischen oder kosmetischen Zusammensetzung erhalten ist.

**Claims**

1. An isolated polypeptide having a glycosyltransferase activity apt to form dextrans with $\alpha(1\rightarrow2)$ side-chains, from saccharose, $\alpha$-D-fluoro-glucose, para-nitrophenyl-$\alpha$-D-glucopyranoside, $\alpha$-D-glucopyranoside-$\alpha$-D-sorbofuranoside, or 4-O-$\alpha$-D-galactopyranosylsucrose, said polypeptide being **characterized**:

a. **in that** it comprises the SEQ ID NR 2; or
b. **in that** it has a structure made up, from the polypeptide's amino end, of a signal sequence, a variable region, a first catalytic domain, a glucan-binding domain and a second catalytic domain, in which:

i. the sequences of the first and second catalytic domains of said polypeptide have a similarity percentage with the SEQ ID NR: 1 of between 65% and 100%, the catalytic triad Asp/Glu/Asp in respective positions 230/268/342 of SEQ ID NR: 1 being conserved, and
ii. the glucan-binding domain being over 500 amino-acids in length.

2. The polypeptide according to claim 1, in which the following amino-acids are unchanged compared to the sequence of the SEQ ID NR: 2:

W in positions 425 and 2122,
E in positions 430, 565 and 2127, 2248,
D in positions 487, 489, 527, 638, 2170, 2172, 2210 and 2322,
H in positions 637 and 2321,
Q in positions 1019 and 2694.

3. An isolated nucleic acid coding for an enzyme having a glycosyltransferase activity apt to form dextrans with $\alpha(1\rightarrow2)$ side-chains from saccharose, $\alpha$-D-fluoro-glucose, para-nitrophenyl-$\alpha$-D-glucopyranoside, $\alpha$-D-glucopyranoside-$\alpha$-D-sorbofuranoside, or 4-O-$\alpha$-D-galactopyranosylsucrose, said nucleic acid being **characterized**:

a. **in that** it comprises or consists in the SEQ ID NR 4; or
b. **in that** it comprises, from the 5'-end to the 3'-end, a sequence coding a signal peptide, a variable sequence, a sequence coding a first catalytic domain, a sequence coding a glucan-binding domain and a sequence coding a second catalytic domain, in which:

i. the two nucleic acid sequences coding the catalytic domains have at least 80% sequence identity with the SEQ ID NR: 3, the coding of the catalytic triad Asp/Glu/Asp coded by the GAT, GAA and GAT triplets in positions 6628/6742/6964 of SEQ ID NR: 4 being conserved, and
ii. the nucleic acid sequence coding a glucan-binding domain codes for a glucan-binding domain being over 500 amino-acids in length.

**4.** An isolated nucleic acid according to claim 3, comprising a sequence having at least 80% sequence identity with the sequence coding for a dextran saccharase expressed by the pCR-T7-*dsr*D plasmid deposited at the CNCM on March 15, 2001, under the reference number I-2649, in which the coding of the catalytic triad Asp/Glu/Asp coded by the GAT, GAA and GAT triplets in positions 6628/6742/6964 of SEQ ID NR: 4 is conserved.

**5.** An expression vector comprising the nucleic acid sequence according to any of claim 3 or claim 4.

**6.** The vector according to claim 5, in which the nucleic acid is under the control of a sequence allowing its expression in prokaryote or eukaryote cells.

**7.** Host cell comprising a nucleic acid according to claim 3 or 4 or a vector according to claim 5 or 6 selected in a group comprising *E. coli*, plants, *Lactococcus, Bacillus* and yeasts.

**8.** A cell according to claim 7, **characterized in that** it is an *E. coli* strain deposited at the CNCM on March 15, 2001, under the reference number I-2649.

**9.** A process for producing a dextran saccharase apt to form $\alpha 1 \rightarrow 2$ links, comprising the steps of:

a) inserting a nucleic acid according to any of claims 3 or 4 or a vector according to any of claims 5 or 6 in a host cell according to claim 7;
b) purifying the enzyme from a cell extract.

**10.** Process according to claim 9, in which the host cell is an eukaryote selected from a group comprising yeasts, fungi and plants.

**11.** Process for obtaining an isolated glycosyltransferase apt to form oligosides or dextrans with a content of $\alpha(1 \rightarrow 2)$ links greater than 30%, comprising:

- a step of randomly modifying the sequence SEQ ID NR 4 in order to establish a variant library,
- a step of expressing these modified sequences in an appropriate host cell, one host cell containing one variant,
- a step of screening the host cells expressing an enzyme apt to form more than 30% of $\alpha(1 \rightarrow 2)$ links on an appropriate substrate,
- a step of isolating the one or several improved genes.

**12.** The use of a glycosyltransferase obtained by a process according to any of claim 9 or 10 in the manufacture of a pharmaceutical or a cosmetic composition.

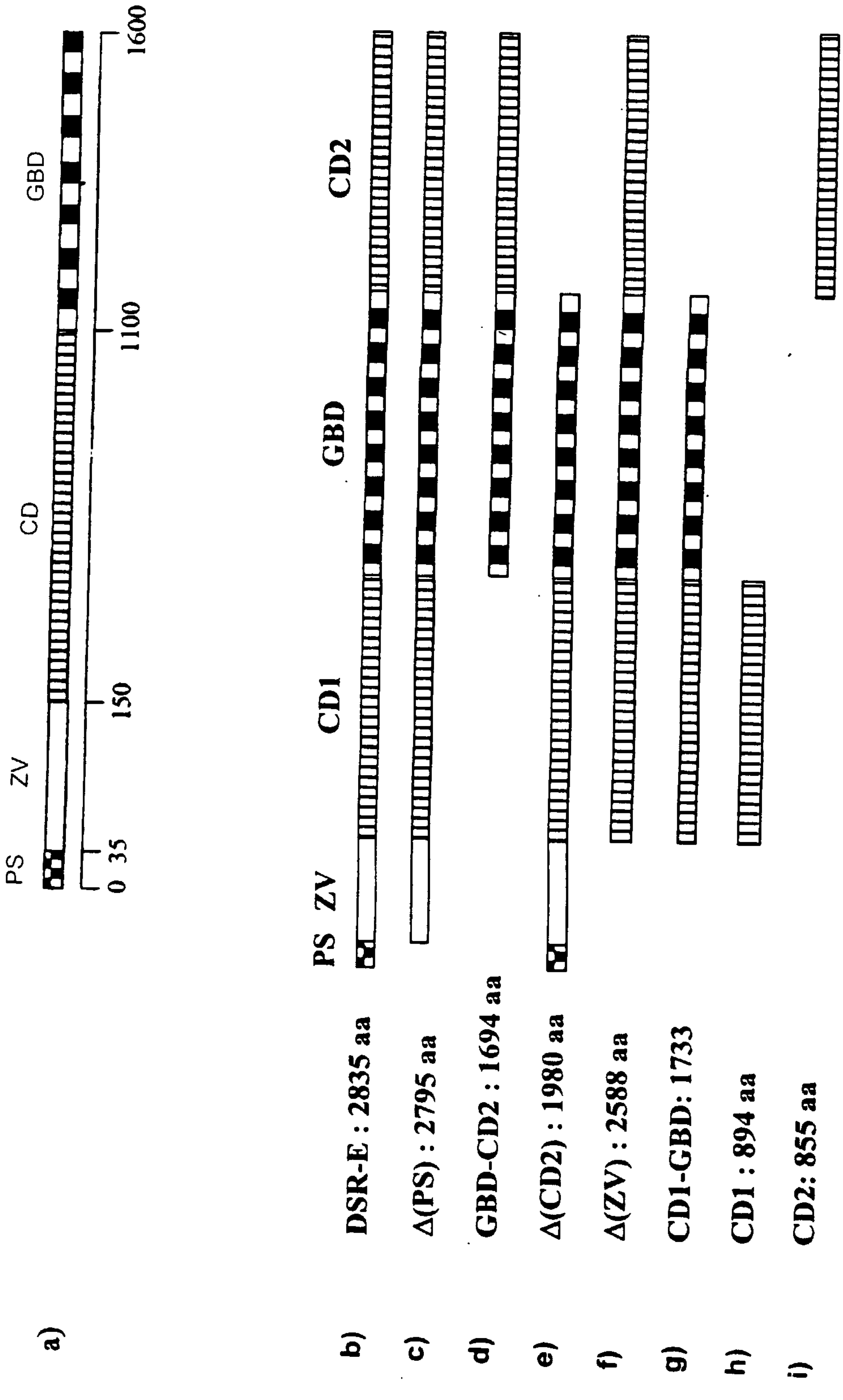
a)
PS
ZV
CD
GBD
0
35
150
1100
1600
PS ZV
CD1
GBD
CD2
b) DSR-E : 2835 aa
c) Δ(PS) : 2795 aa
d) GBD-CD2 : 1694 aa
e) Δ(CD2) : 1980 aa
f) Δ(ZV) : 2588 aa
g) CD1-GBD: 1733
h) CD1 : 894 aa
i) CD2: 855 aa

Fig. 1

Fig. 2

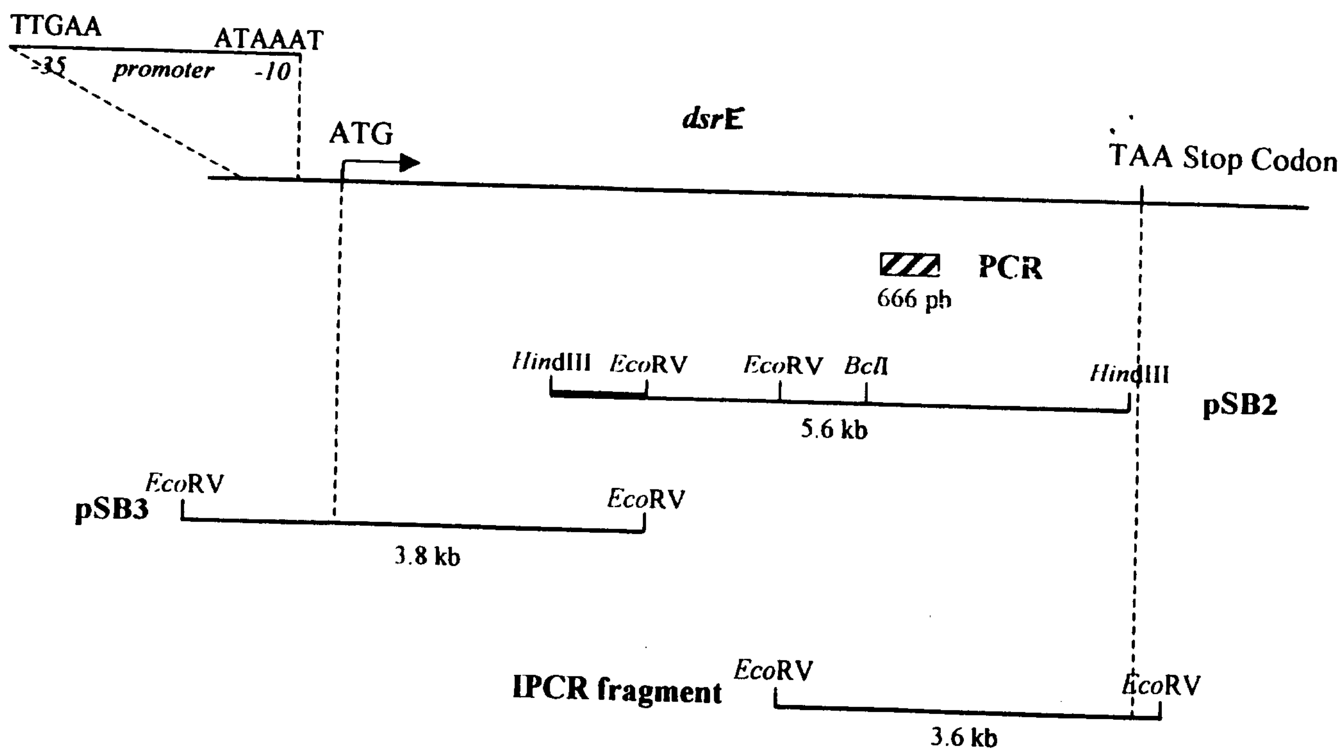


Fig. 3

| | |
|---|---|
| DSR-E | MRDMRVICDRKKLYKSGKVLVTAG-IFALMMFGVTTASVSA |
| DSR-B | MFMIKERNVRKKLYKSGKSWVIGGLILSTIMLSMTATS--- |
| DSR-S | -MPFTEKVMRKKLYKVGKSWVVGG----VCAFALTAS---- |
| ASR | -MKQQETVTRKK-YKSGKVWVAAATAFAVLGVSTVTTVHA- |

# Fig. 4

$^{73}$AAKVVAVATTP-AT
$^{86}$PVADKTVSA
$^{95}$PAADKAVDTTSSTT
$^{109}$PATDKAVDTTP-TT
$^{122}$PAADKAVDTTP-TT
$^{135}$PAADKAVDTTP-TT
$^{148}$PAANKAVDTTP-AT
$^{161}$AATDKAV-ATP-AT
$^{173}$PAADKLANTT--AT
$^{185}$---DKAVATTP-AT
$^{196}$PVANKAA

PAADKAVDTTP-A/T T ← Proposed consensus sequence for S repeat

# Fig. 5

```
                    A                              B
GTFB   341   SAWNSDSEK----PFDDHLQN   402   GGYEFLLANDVDNSNPVVQAEQLN
GTFI   341   PQWNGESEK----PYDDHLQN   404   GGYELLLANDVDNSNPIVQAEQLN
GTFS   327   NQWSIASENETVYPNQDHMQG   388   AGYELLLANDVDNSNPVVQAEQLN
dsrS   444   PQWNETSED----MSNDHLQN   502   GGFELLLANDVDNSNPVVQAEQLN
dsrA   181   PNWNIDSEA----KGDDHLQG   237   GGFELLLANDVDNSNPVVQAEQLN
dsrB   426   PQWNMSSED----PKNDHLQN   484   GGFELLLANDVDNSNPVVQSEQLN
asr    525   ANWNKQTEDEAF-DGLQWLQG   585   KGSEFLLANDIDNSNPIVQAEQLN
CD1    423   ANWNIDSES----KGNDHLQG   478   GGYEMLLANDVDNSNPVVQAEQLN
CD2    2120  FIWNKDSEYHG--GGDAWFQG   2161  NAFDFLLANDVDNSNPVVQAENLN
               *.  :*           :*.         . ::*****:*****:**:*:**

                     C                      D                   E                 F
GTFB   443   ANFDSIRVDAVDNVDADLLQI   484   HLSILEAWSDND   555   YSFIRAHDSEVQDLI   928   DWVPDQMY
GTFI   445   ANFDSIRVDAVDNVDADLLQI   486   HVSIVEAWSDND   557   YSFARAHDSEVQDLI   932   DWVPDQMY
GTFS   429   ANFDGVRVDAVDNVNADLLQI   470   HLSILEAWSGND   540   YVFIRAHDSEVQTRI   915   DLVPNQLY
dsrS   543   ANFDGIRVDAVDNVDADLLQI   584   HLSILEDWSHND   655   YSFVRAHDSEVQTVI   1024  DWVPDQIY
dsrA   278   ANFDGYRVDAVDNVDADLLQI   319   IYQFWKTGEMKI   390   YSFIRAHDSEVQTII   765   DWVPDQIY
dsrB   525   ANFDGIRVDAVDNVDADLLQI   566   HLSILEDWSHND   637   YSFVRAHDSEVQTVI   1005  DWVPDQIY
asr    626   ANFDGIRVDAVDNVDADLLKI   667   HLSILEDWNGKD   759   YSFVRAHDYDAQDPI   1168  DWVPDQIY
CD1    519   ANFDGYRVDAVDNVDADLLQI   560   HISILEDWDNND   631   YAFIRAHDSEVQTVI   1014  DWVPDQIY
CD2    2202  ANFDSIRIDAVDFIHNDTIQR   2243  HISLVEAG----   2315  YSIIHAHDKGVQEKV   2689  DVVDNQVY
             ****. *:**** :. * ::              .: :              * : :***  .*  :          * * :*:*
```

Fig. 6

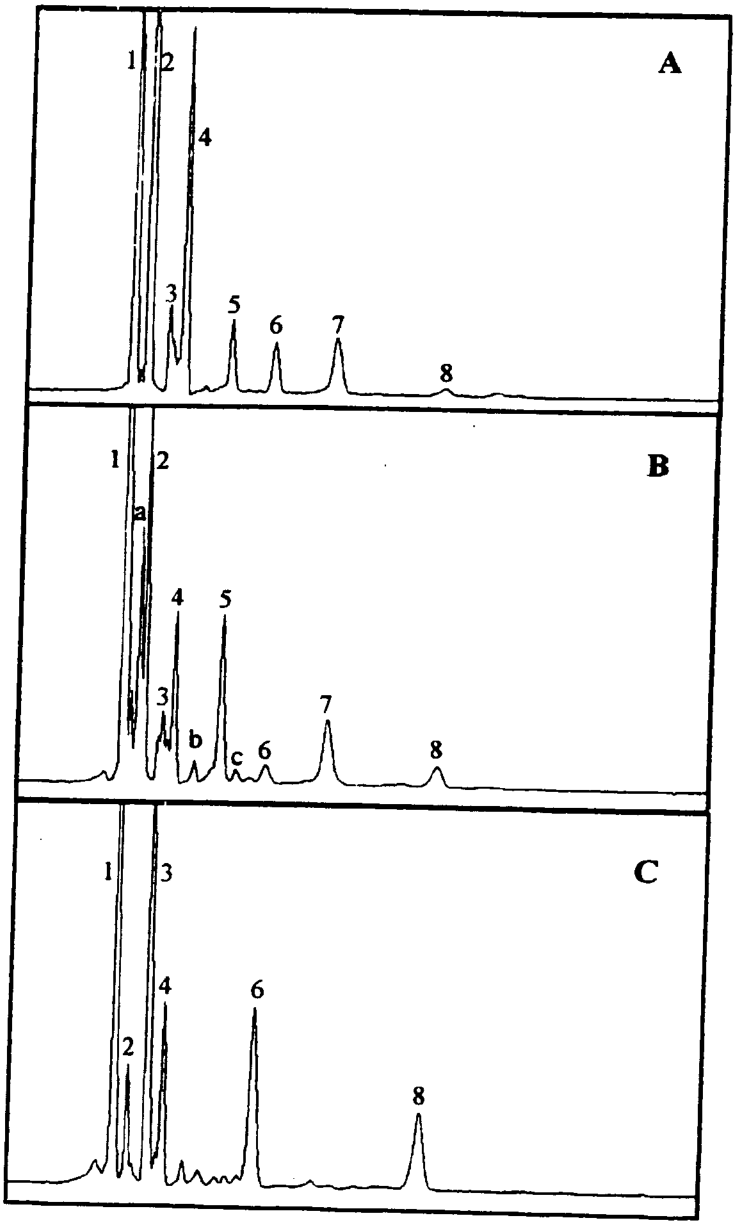

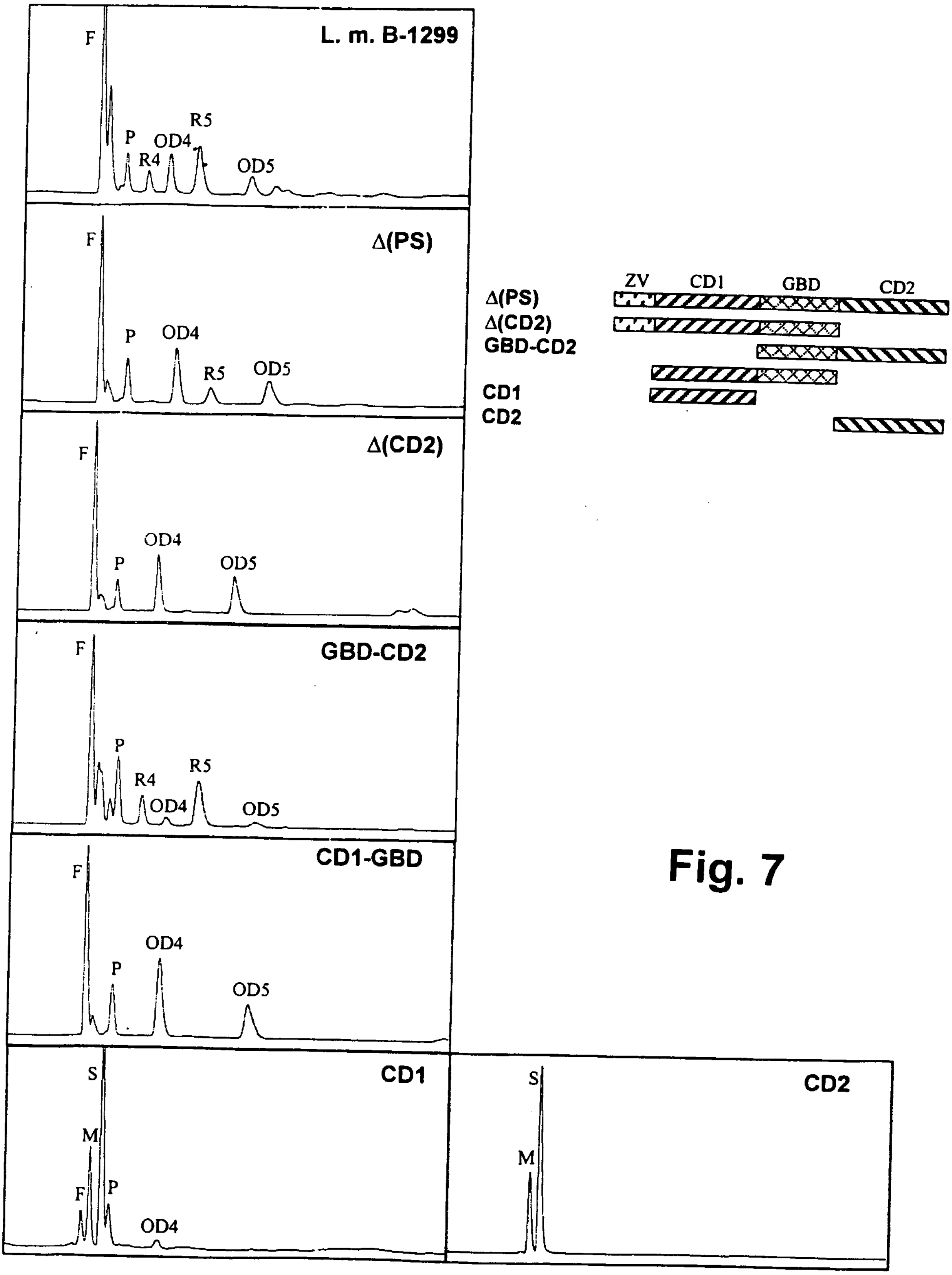
L. m. B-1299
F
P
R4
OD4
R5
OD5
Δ(PS)
Δ(CD2)
GBD-CD2
CD1-GBD
CD1
CD2
S
M
ZV
CD1
GBD
CD2
Δ(PS)
Δ(CD2)
GBD-CD2
CD1
CD2

Fig. 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0325872 B1
- FR 0103631
- WO 8912386 A
- WO 0103631 A


### Littérature non-brevet citée dans la description


- **THOMPSON et al.** *Nucleic Acid Research,* 1994, vol. 22, 4673-4680
- **MONCHOIS V. ; WILLEMOT R.M. ; MONSAN P.** Glucansucrases : mechanism of action and structure-function relation-ships. *FEMS microbiol. Rev.,* 1999, vol. 23, 131-151
- **DOLS M. ; REMAUD-SIMEON M. ; WILLEMOT R.M. ; VIGNON M.R. ; MONSAN P.F.** Structural characterization of the maltose acceptor-products synthesised by Leuconostoc mesenteroides NRRL B-1299 dextransucrase. *Carbohydrate Research,* 1998, vol. 305, 549-559
- **ARNOLD F.H.** *Nature,* 2001, vol. 409 (6817), 253
- **MONCHOIS V. ; VIGNON M. ; RUSSEL R.R.B.** Isolation of key amino-acid residues at the N-terminal end of the core region of Streptococcus downei glucansucrase GTF-I. *Appl. Microbiol. Biotechnol.,* 1999, vol. 52, 660-665
- **ARGUELLO-MORALES M.A. ; REMAUD-SIMEON M. ; PIZZUT S. ; SARÇABAL P. ; WILLEMOT R.M. ; MONSAN P.** Sequence analysis of the gene encoding alternansucrase, a sucrose glucosyltransferase from Leuconostoc mesenteroides NRRL B-1355. *FEMS Microb. Lett.,* 2000, vol. 182, 81-85
- **DEVULAPALLE K.S. ; GOODMAN S. ; GAO Q. ; HEMSLEY A. ; MOOSER G.** Knowledge-based model of a glusocyltransferase from oral bacterial group of mutants streptococci. *Protein Sci.,* 1997, vol. 6, 2489-2493
- **KATO C. ; KURAMITSU H.K.** Carboxy-terminal deletion analysis of the Streptococcus mutans glucosyl-transferase-I enzyme. *FEMS Microbiol. Lett.,* 1990, vol. 72, 299-302
- **LIS M. ; SHIROZA T. ; KURAMITSU H.K.** Role of the C-terminal direct repeating units of the Streptococcus mutans glucosyltransferase-S in glucan binding. *Appl. Env. Microbiol.,* 1995, vol. 61, 2040-2042
- **MONCHOIS V. ; REMAUD-SIMEON M. ; RUSSEL R.R.B. ; MONSAN P. ; WILLEMOT R.M.** Characterization of Leuconostoc mesenteroides NRRL B-512F dextransucrase (DSR-S) and identification of amino-acid residues playing a key role in enzyme activity. *Appl. Microbiol. Biotechnol.,* 1997, vol. 48, 465-472

LISTE DE SEQUENCES

<110> Centre National de la Recherche Scientifique

<120> MOLECULE D'ACIDES NUCLEIQUES CODANT UNE DEXTRANE-SACCHARASE CATALYSANT LA SYNTHESE DE DEXTRANE PORTANT DES RAMIFICATIONS DE TYPE ALPHA-1,2 OSIDIQUES

<130> B4787A (INPI) CNRS/INSA TOULOUSE

<140> xxxxxxx
<141> 2001-12-19

<150> 0103631
<151> 2001-03-16 .

<160> 17

<170> PatentIn Ver. 2.1

<210> 1
<211> 855
<212> PRT
<213> Acides aminés (domaine catalytique n° 2)

<400> 1

```
Asp Met Ser Thr Asn Ala Phe Ser Thr Lys Asn Val Ala Phe Asn His
 1               5                  10                  15

Asp Ser Ser Ser Phe Asp His Thr Val Asp Gly Phe Leu Thr Ala Asp
            20                  25                  30

Thr Trp Tyr Arg Pro Lys Ser Ile Leu Ala Asn Gly Thr Thr Trp Arg
        35                  40                  45

Asp Ser Thr Asp Lys Asp Met Arg Pro Leu Ile Thr Val Trp Trp Pro
    50                  55                  60

Asn Lys Asn Val Gln Val Asn Tyr Leu Asn Phe Met Lys Ala Asn Gly
65                  70                  75                  80

Leu Leu Thr Thr Ala Ala Gln Tyr Thr Leu His Ser Asp Gln Tyr Asp
                85                  90                  95

Leu Asn Gln Ala Ala Gln Asp Val Gln Val Ala Ile Glu Arg Arg Ile
            100                 105                 110

Ala Ser Glu His Gly Thr Asp Trp Leu Gln Lys Leu Leu Phe Glu Ser
        115                 120                 125

Gln Asn Asn Asn Pro Ser Phe Val Lys Gln Gln Phe Ile Trp Asn Lys
    130                 135                 140

Asp Ser Glu Tyr His Gly Gly Gly Asp Ala Trp Phe Gln Gly Gly Tyr
145                 150                 155                 160
```

```
Leu Lys Tyr Gly Asn Asn Pro Leu Thr Pro Thr Thr Asn Ser Asp Tyr
                165                 170                 175

Arg Gln Pro Gly Asn Ala Phe Asp Phe Leu Leu Ala Asn Asp Val Asp
            180                 185                 190

Asn Ser Asn Pro Val Val Gln Ala Glu Asn Leu Asn Trp Leu His Tyr
        195                 200                 205

Leu Met Asn Phe Gly Thr Ile Thr Ala Gly Gln Asp Asp Ala Asn Phe
    210                 215                 220

Asp Ser Ile Arg Ile Asp Ala Val Asp Phe Ile His Asn Asp Thr Ile
225                 230                 235                 240

Gln Arg Thr Tyr Asp Tyr Leu Arg Asp Ala Tyr Gln Val Gln Gln Ser
                245                 250                 255

Glu Ala Lys Ala Asn Gln His Ile Ser Leu Val Glu Ala Gly Leu Asp
            260                 265                 270

Ala Gly Thr Ser Thr Ile His Asn Asp Ala Leu Ile Glu Ser Asn Leu
        275                 280                 285

Arg Glu Ala Ala Thr Leu Ser Leu Thr Asn Glu Pro Gly Lys Asn Lys
    290                 295                 300

Pro Leu Thr Asn Met Leu Gln Asp Val Asp Gly Gly Thr Leu Ile Thr
305                 310                 315                 320

Asp His Thr Gln Asn Ser Thr Glu Asn Gln Ala Thr Pro Asn Tyr Ser
                325                 330                 335

Ile Ile His Ala His Asp Lys Gly Val Gln Glu Lys Val Gly Ala Ala
            340                 345                 350

Ile Thr Asp Ala Thr Gly Ala Asp Trp Thr Asn Phe Thr Asp Glu Gln
        355                 360                 365

Leu Lys Ala Gly Leu Glu Leu Phe Tyr Lys Asp Gln Arg Ala Thr Asn
    370                 375                 380

Lys Lys Tyr Asn Ser Tyr Asn Ile Pro Ser Ile Tyr Ala Leu Met Leu
385                 390                 395                 400

Thr Asn Lys Asp Thr Val Pro Arg Met Tyr Tyr Gly Asp Met Tyr Gln
                405                 410                 415

Asp Asp Gly Gln Tyr Met Ala Asn Lys Ser Ile Tyr Tyr Asp Ala Leu
            420                 425                 430

Val Ser Leu Met Thr Ala Arg Lys Ser Tyr Val Ser Gly Gly Gln Thr
        435                 440                 445

Met Ser Val Asp Asn His Gly Leu Leu Lys Ser Val Arg Phe Gly Lys
    450                 455                 460

Asp Ala Met Thr Ala Asn Asp Leu Gly Thr Ser Ala Thr Arg Thr Glu
465                 470                 475                 480
```

```
Gly Leu Gly Val Ile Ile Gly Asn Asp Pro Lys Leu Gln Leu Asn Asp
                485                 490                 495

Ser Asp Lys Val Thr Leu Asp Met Gly Ala Ala His Lys Asn Gln Lys
            500                 505                 510

Tyr Arg Ala Val Ile Leu Thr Thr Arg Asp Gly Leu Ala Thr Phe Asn
        515                 520                 525

Ser Asp Gln Ala Pro Thr Ala Trp Thr Asn Asp Gln Gly Thr Leu Thr
    530                 535                 540

Phe Ser Asn Gln Glu Ile Asn Gly Gln Asp Asn Thr Gln Ile Arg Gly
545                 550                 555                 560

Val Ala Asn Pro Gln Val Ser Gly Tyr Leu Ala Val Trp Val Pro Val
                565                 570                 575

Gly Ala Ser Asp Asn Gln Asp Ala Arg Thr Ala Ala Thr Thr Thr Glu
            580                 585                 590

Asn His Asp Gly Lys Val Leu His Ser Asn Ala Ala Leu Asp Ser Asn
        595                 600                 605

Leu Ile Tyr Glu Gly Phe Ser Asn Phe Gln Pro Lys Ala Thr Thr His
    610                 615                 620

Asp Glu Leu Thr Asn Val Val Ile Ala Lys Asn Ala Asp Val Phe Asn
625                 630                 635                 640

Asn Trp Gly Ile Thr Ser Phe Glu Met Ala Pro Gln Tyr Arg Ser Ser
                645                 650                 655

Gly Asp His Thr Phe Leu Asp Ser Thr Ile Asp Asn Gly Tyr Ala Phe
            660                 665                 670

Thr Asp Arg Tyr Asp Leu Gly Phe Asn Thr Pro Thr Lys Tyr Gly Thr
        675                 680                 685

Asp Gly Asp Leu Arg Ala Thr Ile Gln Ala Leu His His Ala Asn Met
    690                 695                 700

Gln Val Met Ala Asp Val Val Asp Asn Gln Val Tyr Asn Leu Pro Gly
705                 710                 715                 720

Lys Glu Val Val Ser Ala Thr Arg Ala Gly Val Tyr Gly Asn Asp Asp
                725                 730                 735

Ala Thr Gly Phe Gly Thr Gln Leu Tyr Val Thr Asn Ser Val Gly Gly
            740                 745                 750

Gly Gln Tyr Gln Glu Lys Tyr Ala Gly Gln Tyr Leu Glu Ala Leu Lys
        755                 760                 765

Ala Lys Tyr Pro Asp Leu Phe Glu Gly Lys Ala Tyr Asp Tyr Trp Tyr
    770                 775                 780

Lys Asn Tyr Ala Asn Asp Gly Ser Asn Pro Tyr Tyr Thr Leu Ser His
785                 790                 795                 800
```

```
Gly Asp Arg Glu Ser Ile Pro Ala Asp Val Ala Ile Lys Gln Trp Ser
                805                 810                 815

Ala Lys Tyr Met Asn Gly Thr Asn Val Leu Gly Asn Gly Met Gly Tyr
            820                 825                 830

Val Leu Lys Asp Trp His Asn Gly Gln Tyr Phe Lys Leu Asp Gly Asp
        835                 840                 845

Lys Ser Thr Leu Pro Gln Ile
    850                 855
```

<210> 2
<211> 2835
<212> PRT
<213> Acides aminés (protéine complète DSR-E)

<400> 2

```
Met Arg Asp Met Arg Val Ile Cys Asp Arg Lys Lys Leu Tyr Lys Ser
 1               5                  10                  15

Gly Lys Val Leu Val Thr Ala Gly Ile Phe Ala Leu Met Met Phe Gly
            20                  25                  30

Val Thr Thr Ala Ser Val Ser Ala Asn Thr Ile Ala Val Asp Thr Asn
        35                  40                  45

His Ser Arg Thr Ser Ala Gln Ile Asn Lys Ser Ala Val Asp Lys Val
    50                  55                  60

Asn Asp Asp Lys Thr Thr Leu Gly Ala Ala Lys Val Val Ala Val Ala
 65                 70                  75                  80

Thr Thr Pro Ala Thr Pro Val Ala Asp Lys Thr Val Ser Ala Pro Ala
                85                  90                  95

Ala Asp Lys Ala Val Asp Thr Thr Ser Ser Thr Thr Pro Ala Thr Asp
            100                 105                 110

Lys Ala Val Asp Thr Thr Pro Thr Thr Pro Ala Ala Asp Lys Ala Val
        115                 120                 125

Asp Thr Thr Pro Thr Thr Pro Ala Ala Asp Lys Ala Val Asp Thr Thr
    130                 135                 140

Pro Thr Thr Pro Ala Ala Asn Lys Ala Val Asp Thr Thr Pro Ala Thr
145                 150                 155                 160

Ala Ala Thr Asp Lys Ala Val Ala Thr Pro Ala Thr Pro Ala Ala Asp
                165                 170                 175

Lys Leu Ala Asn Thr Thr Pro Ala Thr Asp Lys Ala Val Ala Thr Thr
            180                 185                 190

Pro Ala Thr Pro Val Ala Asn Lys Ala Ala Asp Thr Ser Ser Ile His
        195                 200                 205

Asp Gln Pro Leu Asp Thr Asn Val Pro Thr Asp Lys Ser Ala Asn Leu
    210                 215                 220
```

```
Val Ser Thr Thr Gln Lys Ser Thr Asp Asn Gln Gln Val Lys Ser Thr
225                 230                 235                 240

Glu Thr Ser His Leu Gln Glu Ile Asn Gly Lys Thr Tyr Phe Leu Asp
                245                 250                 255

Asp Asn Gly Gln Val Lys Lys Asn Phe Thr Ala Ile Ile Asp Gly Lys
            260                 265                 270

Val Leu Tyr Phe Asp Lys Thr Ser Gly Glu Leu Thr Ala Asn Ala Pro
        275                 280                 285

Gln Val Thr Lys Gly Leu Val Asn Ile Asp Asn Ala His Asn Ala Ala
    290                 295                 300

His Asp Leu Thr Ala Asp Asn Phe Thr Asn Val Asp Gly Tyr Leu Thr
305                 310                 315                 320

Ala Asn Ser Trp Tyr Arg Pro Lys Asp Ile Leu Lys Asn Gly Thr Thr
                325                 330                 335

Trp Thr Pro Thr Thr Ala Glu Asp Phe Arg Pro Leu Leu Met Ser Trp
            340                 345                 350

Trp Pro Asp Lys Asn Thr Gln Val Ala Tyr Leu Gln Tyr Met Gln Ser
        355                 360                 365

Val Gly Met Leu Pro Asp Asp Val Lys Val Ser Asn Asp Asp Asn Met
    370                 375                 380

Ser Thr Leu Thr Asp Ala Ala Met Thr Val Gln Lys Asn Ile Glu Ser
385                 390                 395                 400

Arg Ile Gly Val Ser Gly Lys Thr Asp Trp Leu Lys Gln Asp Met Asn
                405                 410                 415

Lys Leu Ile Asp Ser Gln Ala Asn Trp Asn Ile Asp Ser Glu Ser Lys
            420                 425                 430

Gly Asn Asp His Leu Gln Gly Gly Ala Leu Leu Tyr Val Asn Asp Asp
        435                 440                 445

Lys Thr Pro Asn Ala Asn Ser Asp Tyr Arg Leu Leu Asn Arg Thr Pro
    450                 455                 460

Thr Asn Gln Thr Gly Gln Ile Thr Asp Pro Ser Lys Gln Gly Gly Tyr
465                 470                 475                 480

Glu Met Leu Leu Ala Asn Asp Val Asp Asn Ser Asn Pro Val Val Gln
                485                 490                 495

Ala Glu Gln Leu Asn Trp Leu His Tyr Met Met Asn Ile Gly Thr Ile
            500                 505                 510

Ala Gln Asn Asp Pro Thr Ala Asn Phe Asp Gly Tyr Arg Val Asp Ala
        515                 520                 525

Val Asp Asn Val Asp Ala Asp Leu Leu Gln Ile Ala Gly Asp Tyr Phe
    530                 535                 540
```

```
Lys Ala Ala Tyr Gly Thr Gly Lys Thr Glu Ala Asn Ala Asn Asn His
545                 550                 555                 560

Ile Ser Ile Leu Glu Asp Trp Asp Asn Asn Asp Ser Ala Tyr Ile Lys
                565                 570                 575

Ala His Gly Asn Asn Gln Leu Thr Met Asp Phe Pro Ala His Leu Ala
            580                 585                 590

Leu Lys Tyr Ala Leu Asn Met Pro Leu Ala Ala Gln Ser Gly Leu Glu
        595                 600                 605

Pro Leu Ile Asn Thr Ser Leu Val Lys Arg Gly Lys Asp Ala Thr Glu
    610                 615                 620

Asn Glu Ala Gln Pro Asn Tyr Ala Phe Ile Arg Ala His Asp Ser Glu
625                 630                 635                 640

Val Gln Thr Val Ile Ala Gln Ile Ile Lys Asp Lys Ile Asn Thr Lys
                645                 650                 655

Ser Asp Gly Leu Thr Val Thr Pro Asp Glu Ile Lys Gln Ala Phe Thr
            660                 665                 670

Ile Tyr Asn Ala Asp Glu Leu Lys Ala Asp Lys Glu Tyr Thr Ala Tyr
        675                 680                 685

Asn Ile Pro Ala Ser Tyr Ala Val Leu Leu Thr Asn Lys Asp Thr Val
    690                 695                 700

Pro Arg Val Tyr Tyr Gly Asp Leu Phe Ser Asp Asp Gly Gln Tyr Met
705                 710                 715                 720

Ser Gln Lys Ser Pro Tyr Tyr Asp Ala Ile Thr Ser Leu Leu Lys Ser
                725                 730                 735

Arg Ile Lys Tyr Val Ala Gly Gly Gln Ser Met Asn Met Thr Tyr Leu
            740                 745                 750

His Glu Cys Phe Asp Pro Ala Lys Asn Glu Thr Lys Pro Gln Gly Val
        755                 760                 765

Leu Thr Ser Val Arg Tyr Gly Lys Gly Ala Met Thr Ala Asp Asp Leu
    770                 775                 780

Gly Asn Ser Asp Thr Arg Gln Gln Gly Ile Gly Leu Val Ile Asn Asn
785                 790                 795                 800

Lys Pro Phe Leu Asn Leu Asn Asp Asp Glu Gln Ile Val Leu Asn Met
                805                 810                 815

Gly Ala Ala His Lys Asn Gln Ala Tyr Arg Pro Leu Met Leu Thr Thr
            820                 825                 830

Lys Ser Gly Leu Gln Ile Tyr Asp Lys Asp Ala Gly Ala Pro Val Val
        835                 840                 845

Tyr Thr Asn Asp Ala Gly Gln Leu Ile Phe Lys Ser Asp Met Val Tyr
    850                 855                 860
```

Gly Val Ser Asn Pro Gln Val Ser Gly Tyr Phe Ala Ala Trp Val Pro
865 870 875 880

Val Gly Ala Ser Asp Ser Gln Asp Ala Arg Thr Gln Ser Ser Gln Ser
885 890 895

Glu Thr Lys Asp Gly Asp Val Tyr His Ser Asn Ala Ala Leu Asp Ser
900 905 910

Asn Val Ile Tyr Glu Gly Phe Ser Asn Phe Gln Ala Met Pro Glu Lys
915 920 925

Asn Asp Asp Phe Thr Asn Val Lys Ile Ala Gln Asn Ala Lys Leu Phe
930 935 940

Lys Asp Leu Gly Ile Thr Ser Phe Glu Leu Ala Pro Gln Tyr Arg Ser
945 950 955 960

Ser Thr Asp Asn Ser Phe Leu Asp Ser Val Ile Gln Asn Gly Tyr Ala
965 970 975

Phe Thr Asp Arg Tyr Asp Val Gly Tyr Asn Thr Pro Thr Lys Tyr Gly
980 985 990

Thr Val Asp Gln Leu Leu Asp Ser Leu Arg Ala Leu His Ala Gln Gly
995 1000 1005

Ile Gln Ala Ile Asn Asp Trp Val Pro Asp Gln Ile Tyr Asn Leu Pro
1010 1015 1020

Gly Glu Gln Ile Val Thr Ala Val Arg Thr Asn Gly Ser Gly Lys Tyr
1025 1030 1035 1040

Asp Tyr Asp Ser Val Ile Asn Asn Thr Leu Tyr Asp Ser Arg Thr Val
1045 1050 1055

Gly Gly Gly Glu Tyr Gln Glu Lys Phe Gly Gly Leu Phe Leu Asp Gln
1060 1065 1070

Leu Lys Lys Asp Tyr Pro Ser Leu Phe Glu Thr Lys Gln Ile Ser Thr
1075 1080 1085

Asn Gln Pro Met Asn Pro Asp Val Lys Ile Lys Glu Trp Ser Ala Lys
1090 1095 1100

Tyr Phe Asn Gly Ser Asn Ile Gln Gly Arg Gly Ala Trp Tyr Val Leu
1105 1110 1115 1120

Lys Asp Trp Ala Thr Asn Gln Tyr Phe Asn Val Ser Ser Asp Asn Gly
1125 1130 1135

Phe Leu Pro Lys Gln Leu Leu Gly Glu Lys Thr Ser Thr Gly Phe Ile
1140 1145 1150

Thr Glu Asn Gly Lys Thr Ser Phe Tyr Ser Thr Ser Gly Tyr Gln Ala
1155 1160 1165

Lys Asp Thr Phe Ile Gln Asp Gly Thr Asn Trp Tyr Tyr Phe Asp Asn
1170 1175 1180

```
Ala Gly Tyr Met Leu Thr Gly Lys Gln Asn Ile His Asp Lys Asn Tyr
1185                1190                1195                1200

Tyr Phe Leu Pro Asn Gly Val Glu Leu Gln Asp Ala Tyr Leu Phe Asp
               1205                1210                1215

Gly Asn Gln Glu Phe Tyr Tyr Asn Lys Ala Gly Glu Gln Val Met Asn
           1220                1225                1230

Gln Tyr Tyr Gln Asp Ser Gln Asn Gln Trp His Tyr Phe Phe Glu Asn
       1235                1240                1245

Gly Arg Met Ala Ile Gly Leu Thr Glu Val Pro Asn Ala Asp Gly Thr
   1250                1255                1260

His Val Thr Gln Tyr Phe Asp Ala Asn Gly Val Gln Ile Lys Gly Thr
1265                1270                1275                1280

Ala Ile Lys Asp Gln Asn Asn Gln Leu Arg Tyr Phe Asp Glu Ala Thr
               1285                1290                1295

Gly Asn Met Val Val Asn Ser Trp Gly Gln Leu Ala Asp Lys Ser Trp
           1300                1305                1310

Leu Tyr Leu Asn Ala Gln Gly Val Ala Val Thr Gly Asn Gln Lys Ile
       1315                1320                1325

Asp Gly Glu Glu Tyr Tyr Phe Asn Ala Asp Gly Lys Gln Val Lys Gly
   1330                1335                1340

Asn Ala Ile Ile Asp Asn Asn Gly Asp Gln Arg Tyr Tyr Asp Gly Asp
1345                1350                1355                1360

Lys Gly Val Met Val Val Asn Ser Trp Gly Glu Leu Pro Asp Gly Ser
               1365                1370                1375

Trp Leu Tyr Leu Asn Asp Lys Gly Ile Ala Val Thr Gly Arg Gln Val
           1380                1385                1390

Ile Asn Asn Gln Val Asn Phe Phe Gly Asn Asp Gly Lys Gln Ile Lys
       1395                1400                1405

Asp Ala Phe Lys Leu Leu Ser Asp Gly Ser Trp Val Tyr Leu Asp Asp
   1410                1415                1420

Lys Gly Leu Ile Thr Thr Gly Ala Lys Val Ile Asn Gly Leu Asn Met
1425                1430                1435                1440

Phe Phe Asp Lys Asp Gly His Gln Ile Lys Gly Asp Ala Ser Thr Asp
               1445                1450                1455

Ala Asn Gly Lys Arg His Tyr Tyr Asp Lys Asn Asp Gly His Leu Val
           1460                1465                1470

Thr Asn Ser Trp Gly Glu Leu Pro Asp Gly Ser Trp Leu Tyr Leu Glu
       1475                1480                1485

Glu Gln Gly Asp Ala Val Thr Gly Gln Arg Val Ile Asp Gly Lys Thr
   1490                1495                1500
```

Arg Tyr Phe Asp Glu Asp Gly Lys Gln Ile Lys Asn Ser Leu Lys Thr
1505 1510 1515 1520

Leu Ala Asn Gly Asp Lys Ile Tyr Leu Asp Gly Asp Gly Val Ala Ala
1525 1530 1535

Thr Gly Leu Gln His Val Gly Asp Lys Ile Met Tyr Phe Asp Glu Asp
1540 1545 1550

Gly Lys Gln Val Val Gly Lys Phe Val Ser Ala Lys Asp Gly Ser Trp
1555 1560 1565

Tyr Tyr Leu Asn Gln Asp Gly Val Ala Ala Val Gly Pro Ser Ser Ile
1570 1575 1580

Asn Gly Gln Ser Leu Tyr Phe Asp Gln Asp Gly Lys Gln Val Lys Tyr
1585 1590 1595 1600

Asn Glu Val Arg Asn Ser Asp Gly Thr Thr Asn Tyr Tyr Thr Gly Leu
1605 1610 1615

Thr Gly Glu Lys Leu Thr Gln Asp Phe Gly Glu Leu Pro Asp Gly Ser
1620 1625 1630

Trp Ile Tyr Leu Asp Ala Gln Gly His Thr Val Thr Gly Ala Gln Ile
1635 1640 1645

Ile Asn Gly Gln Asn Leu Tyr Phe Lys Ala Asp Gly Gln Gln Val Lys
1650 1655 1660

Gly His Ala Tyr Thr Asp Gln Leu Gly His Met Arg Phe Tyr Asp Pro
1665 1670 1675 1680

Asp Ser Gly Asp Met Leu Ser Asn Arg Phe Glu Gln Ile Thr Pro Gly
1685 1690 1695

Val Trp Ala Tyr Phe Gly Ala Asp Gly Val Ala Ile Thr Gly Gln His
1700 1705 1710

Asp Ile Asn Gly Gln Lys Leu Phe Phe Asp Glu Thr Gly Tyr Gln Val
1715 1720 1725

Lys Gly Ser Gln Arg Thr Ile Asp Gly Thr Leu Tyr Ser Phe Asp Ser
1730 1735 1740

Gln Thr Gly Asn Gln Lys Arg Val Gln Thr Thr Leu Leu Pro Gln Ala
1745 1750 1755 1760

Gly His Tyr Ile Thr Lys Asn Gly Asn Asp Trp Gln Tyr Asp Thr Asn
1765 1770 1775

Gly Glu Leu Ala Lys Gly Leu Arg Gln Asp Ser Asn Gly Lys Leu Arg
1780 1785 1790

Tyr Phe Asp Leu Thr Thr Gly Ile Gln Ala Lys Gly Gln Phe Val Thr
1795 1800 1805

Ile Gly Gln Glu Thr Tyr Tyr Phe Ser Lys Asp His Gly Asp Ala Gln
1810 1815 1820

Leu Leu Pro Met Val Thr Glu Gly His Tyr Gly Thr Ile Thr Leu Lys
1825 1830 1835 1840

Gln Gly Gln Asp Thr Lys Thr Ala Trp Val Tyr Arg Asp Gln Asn Asn
1845 1850 1855

Thr Ile Leu Lys Gly Leu Gln Asn Ile Asn Gly Thr Leu Gln Phe Phe
1860 1865 1870

Asp Pro Tyr Thr Gly Glu Gln Leu Lys Gly Gly Val Ala Lys Tyr Asp
1875 1880 1885

Asp Lys Leu Phe Tyr Phe Glu Ser Gly Lys Gly Asn Leu Val Ser Thr
1890 1895 1900

Val Ala Gly Asp Tyr Gln Asp Gly His Tyr Ile Ser Gln Asp Gly Gln
1905 1910 1915 1920

Thr Arg Tyr Ala Asp Lys Gln Asn Gln Leu Val Lys Gly Leu Val Thr
1925 1930 1935

Val Asn Gly Ala Leu Gln Tyr Phe Asp Asn Ala Thr Gly Asn Gln Ile
1940 1945 1950

Lys Asn Gln Gln Val Ile Val Asp Gly Lys Thr Tyr Tyr Phe Asp Asp
1955 1960 1965

Lys Gly Asn Gly Glu Tyr Leu Phe Thr Asn Thr Leu Asp Met Ser Thr
1970 1975 1980

Asn Ala Phe Ser Thr Lys Asn Val Ala Phe Asn His Asp Ser Ser Ser
1985 1990 1995 2000

Phe Asp His Thr Val Asp Gly Phe Leu Thr Ala Asp Thr Trp Tyr Arg
2005 2010 2015

Pro Lys Ser Ile Leu Ala Asn Gly Thr Thr Trp Arg Asp Ser Thr Asp
2020 2025 2030

Lys Asp Met Arg Pro Leu Ile Thr Val Trp Trp Pro Asn Lys Asn Val
2035 2040 2045

Gln Val Asn Tyr Leu Asn Phe Met Lys Ala Asn Gly Leu Leu Thr Thr
2050 2055 2060

Ala Ala Gln Tyr Thr Leu His Ser Asp Gln Tyr Asp Leu Asn Gln Ala
2065 2070 2075 2080

Ala Gln Asp Val Gln Val Ala Ile Glu Arg Arg Ile Ala Ser Glu His
2085 2090 2095

Gly Thr Asp Trp Leu Gln Lys Leu Leu Phe Glu Ser Gln Asn Asn Asn
2100 2105 2110

Pro Ser Phe Val Lys Gln Gln Phe Ile Trp Asn Lys Asp Ser Glu Tyr
2115 2120 2125

His Gly Gly Gly Asp Ala Trp Phe Gln Gly Gly Tyr Leu Lys Tyr Gly
2130 2135 2140

Asn Asn Pro Leu Thr Pro Thr Thr Asn Ser Asp Tyr Arg Gln Pro Gly
2145                2150                2155                2160

Asn Ala Phe Asp Phe Leu Leu Ala Asn Asp Val Asp Asn Ser Asn Pro
                2165                2170                2175

Val Val Gln Ala Glu Asn Leu Asn Trp Leu His Tyr Leu Met Asn Phe
            2180                2185                2190

Gly Thr Ile Thr Ala Gly Gln Asp Asp Ala Asn Phe Asp Ser Ile Arg
        2195                2200                2205

Ile Asp Ala Val Asp Phe Ile His Asn Asp Thr Ile Gln Arg Thr Tyr
    2210                2215                2220

Asp Tyr Leu Arg Asp Ala Tyr Gln Val Gln Gln Ser Glu Ala Lys Ala
2225                2230                2235                2240

Asn Gln His Ile Ser Leu Val Glu Ala Gly Leu Asp Ala Gly Thr Ser
                2245                2250                2255

Thr Ile His Asn Asp Ala Leu Ile Glu Ser Asn Leu Arg Glu Ala Ala
            2260                2265                2270

Thr Leu Ser Leu Thr Asn Glu Pro Gly Lys Asn Lys Pro Leu Thr Asn
        2275                2280                2285

Met Leu Gln Asp Val Asp Gly Gly Thr Leu Ile Thr Asp His Thr Gln
    2290                2295                2300

Asn Ser Thr Glu Asn Gln Ala Thr Pro Asn Tyr Ser Ile Ile His Ala
2305                2310                2315                2320

His Asp Lys Gly Val Gln Glu Lys Val Gly Ala Ala Ile Thr Asp Ala
                2325                2330                2335

Thr Gly Ala Asp Trp Thr Asn Phe Thr Asp Glu Gln Leu Lys Ala Gly
            2340                2345                2350

Leu Glu Leu Phe Tyr Lys Asp Gln Arg Ala Thr Asn Lys Lys Tyr Asn
        2355                2360                2365

Ser Tyr Asn Ile Pro Ser Ile Tyr Ala Leu Met Leu Thr Asn Lys Asp
    2370                2375                2380

Thr Val Pro Arg Met Tyr Tyr Gly Asp Met Tyr Gln Asp Asp Gly Gln
2385                2390                2395                2400

Tyr Met Ala Asn Lys Ser Ile Tyr Tyr Asp Ala Leu Val Ser Leu Met
                2405                2410                2415

Thr Ala Arg Lys Ser Tyr Val Ser Gly Gly Gln Thr Met Ser Val Asp
            2420                2425                2430

Asn His Gly Leu Leu Lys Ser Val Arg Phe Gly Lys Asp Ala Met Thr
        2435                2440                2445

Ala Asn Asp Leu Gly Thr Ser Ala Thr Arg Thr Glu Gly Leu Gly Val
    2450                2455                2460

```
Ile Ile Gly Asn Asp Pro Lys Leu Gln Leu Asn Asp Ser Asp Lys Val
2465                2470                2475                2480

Thr Leu Asp Met Gly Ala Ala His Lys Asn Gln Lys Tyr Arg Ala Val
                2485                2490                2495

Ile Leu Thr Thr Arg Asp Gly Leu Ala Thr Phe Asn Ser Asp Gln Ala
            2500                2505                2510

Pro Thr Ala Trp Thr Asn Asp Gln Gly Thr Leu Thr Phe Ser Asn Gln
        2515                2520                2525

Glu Ile Asn Gly Gln Asp Asn Thr Gln Ile Arg Gly Val Ala Asn Pro
    2530                2535                2540

Gln Val Ser Gly Tyr Leu Ala Val Trp Val Pro Val Gly Ala Ser Asp
2545                2550                2555                2560

Asn Gln Asp Ala Arg Thr Ala Ala Thr Thr Thr Glu Asn His Asp Gly
                2565                2570                2575

Lys Val Leu His Ser Asn Ala Ala Leu Asp Ser Asn Leu Ile Tyr Glu
            2580                2585                2590

Gly Phe Ser Asn Phe Gln Pro Lys Ala Thr Thr His Asp Glu Leu Thr
        2595                2600                2605

Asn Val Val Ile Ala Lys Asn Ala Asp Val Phe Asn Asn Trp Gly Ile
    2610                2615                2620

Thr Ser Phe Glu Met Ala Pro Gln Tyr Arg Ser Ser Gly Asp His Thr
2625                2630                2635                2640

Phe Leu Asp Ser Thr Ile Asp Asn Gly Tyr Ala Phe Thr Asp Arg Tyr
                2645                2650                2655

Asp Leu Gly Phe Asn Thr Pro Thr Lys Tyr Gly Thr Asp Gly Asp Leu
            2660                2665                2670

Arg Ala Thr Ile Gln Ala Leu His His Ala Asn Met Gln Val Met Ala
        2675                2680                2685

Asp Val Val Asp Asn Gln Val Tyr Asn Leu Pro Gly Lys Glu Val Val
    2690                2695                2700

Ser Ala Thr Arg Ala Gly Val Tyr Gly Asn Asp Asp Ala Thr Gly Phe
2705                2710                2715                2720

Gly Thr Gln Leu Tyr Val Thr Asn Ser Val Gly Gly Gly Gln Tyr Gln
                2725                2730                2735

Glu Lys Tyr Ala Gly Gln Tyr Leu Glu Ala Leu Lys Ala Lys Tyr Pro
            2740                2745                2750

Asp Leu Phe Glu Gly Lys Ala Tyr Asp Tyr Trp Tyr Lys Asn Tyr Ala
        2755                2760                2765

Asn Asp Gly Ser Asn Pro Tyr Tyr Thr Leu Ser His Gly Asp Arg Glu
    2770                2775                2780
```

```
Ser Ile Pro Ala Asp Val Ala Ile Lys Gln Trp Ser Ala Lys Tyr Met
2785                2790                2795                2800

Asn Gly Thr Asn Val Leu Gly Asn Gly Met Gly Tyr Val Leu Lys Asp
                2805                2810                2815

Trp His Asn Gly Gln Tyr Phe Lys Leu Asp Gly Asp Lys Ser Thr Leu
            2820                2825                2830

Pro Gln Ile
        2835
```

<210> 3
<211> 2568
<212> ADN
<213> Nucléotides (domaine catalytique n° 2)

<400> 3

```
gatatgtcta ctaatgcttt ttctaccaaa aatgttgcat tcaatcatga cagtagcagt 60
ttcgaccata ctgttgatgg cttcttgacg gcagatactt ggtatcgacc aaagtcaatt 120
ttggctaacg ggacaacttg gcgtgattcg actgataagg atatgcgacc attaatcact 180
gtttggtggc caaataagaa tgttcaagtc aactacctca acttcatgaa agcaaatggc 240
ttgttgacaa cagcagcaca atacacacta cattcagatc aatatgattt gaaccaagct 300
gcacaagatg ttcaagtggc cattgaaagg cgcattgcgt cagagcatgg cacagactgg 360
ttacagaaat tgttgtttga atcacaaaat aataacccat catttgtgaa gcaacaattc 420
atttggaaca aggattctga atatcatggt ggtggtgatg cttggttcca aggtggttat 480
ctgaagtatg gcaataaccc actcacacca acaactaatt ctgattatcg tcaacctggt 540
aatgcatttg atttcttgct agccaacgac gtggataatt ctaatcctgt tgtgcaagct 600
gaaaacttaa actggttaca ttacttaatg aactttggca ccatcactgc gggtcaagat 660
gacgctaatt ttgatagtat tcgtattgac gctgtcgact ttattcataa tgatacaatc 720
caacgtactt atgattatct tcgtgatgct tatcaagtgc aacaaagtga agccaaagca 780
aaccagcaca tttcattggt tgaagctggc ttagacgcag gtacatcaac gattcataat 840
gatgcgttaa ttgagtcaaa cctccgtgaa gcagcgacat tgtcgttaac aaatgaacct 900
ggtaaaaata aaccattgac gaatatgcta caagacgttg acggcggtac gcttatcacc 960
gaccatacgc agaatagtac agaaaatcag gcgacaccaa actattcaat tattcacgcg 1020
cacgataaag gtgtgcaaga aaaagtaggt gcagccatta ctgatgctac tggtgctgat 1080
tggacgaact ttacagatga acagttaaaa gccggattag agctattcta taaggatcag 1140
cgcgcaacaa acaaaaagta taatagttat aacataccaa gtatttatgc cctgatgttg 1200
acaaacaaag atactgttcc tcgtatgtat tatggggata tgtatcaaga tgacggacag 1260
tatatggcaa acaagagtat ctactatgat gccttagtgt cattaatgac ggctcgtaaa 1320
agctatgtca gcggtggtca aactatgagt gttgacaatc atggtttgtt gaagagtgtc 1380
cgttttggaa aagatgcgat gacagctaat gatttaggta catcagctac gcgtactgag 1440
ggtcttggtg tcattattgg taatgatcca aagttgcaac ttaatgattc ggataaagtg 1500
acactggata tgggtgcagc acataaaaat caaaagtatc gcgcagttat cttaacaaca 1560
cgtgatggtt tggcaacctt taattcagat caagcaccaa cagcttggac aaacgatcaa 1620
ggaacgttaa cattctcaaa tcaagagatt aacgggcaag acaatacaca aattcgtggt 1680
gttgctaatc cgcaagtttc tggttatcta gctgtttggg tgcctgtggg tgcatcagac 1740
aatcaagatg cccgtacagc agcaacgaca acagaaaatc atgatggtaa agtattacac 1800
tcgaatgcgg cattagattc taaccttatt tatgaaggtt tctctaactt ccaacctaag 1860
gcaacaacgc atgatgaact tacgaacgtt gtaattgcta aaaatgccga tgtcttcaat 1920
aattggggta ttacgagttt tgaaatggca ccacagtacc gttcaagtgg ggaccataca 1980
ttcttggatt caacgattga taatggttat gccttcactg atcgctatga cttaggtttc 2040
aatacaccaa caaagtatgg cactgatggt gatttgcgtg caacgattca agcgctacat 2100
catgctaata tgcaagttat ggctgacgtt gttgataacc aggtctataa cttacctggt 2160
aaagaagttg tttcagcaac acgagcaggt gtttatggta atgacgacgc cacgggcttt 2220
ggaacgcaac tctatgtgac taactccgtt ggtggtggtc aataccaaga gaaatatgct 2280
ggacaatact tagaagctct gaaagcaaag tatccagacc tctttgaggg taaggcctat 2340
gattattggt ataagaacta tgcaaatgat gggtcaaatc cttactatac attgtcacac 2400
ggtgaccgtg aatctatccc agcagatgtt gctattaagc aatggtcagc taagtatatg 2460

aacggcacga acgttttggg caatggtatg ggttatgtat tgaaggattg gcataatggt 2520
caatatttca agcttgatgg tgataaatca acattacctc aaatttaa              2568
```

<210> 4
<211> 8506
<212> ADN
<213> Séquence codant DSR-E

<400> 4

atgagagaca tgagggtaat ttgtgaccgt aaaaaattgt acaaatcggg caaagtacta 60
gtaacagccg gtatttttgc tttgatgatg tttggcgtca caactgctag tgttagtgca 120
aatacgattg cagttgacac gaatcatagc cgtacttcag cacagattaa taagagtgcc 180
gttgataagg ttaatgatga caagactact ttaggagcgg caaaagtagt ggcagtagcc 240
acaacgccag cgacagcggt agcagataaa acagtaagtg cacccgcagc agataaggca 300
gtagatacaa cgtcatcaac gacacctgca acggataagg cagtagatac aacgccaacg 360
acacctgcag cagataaggc agtagataca acgccaacga cacctgcagc agataaggca 420
gtagatacaa cgccaacgac acctgcagca aataaagcag tagatacaac gccagcgacc 480
gctgcaacag ataaggcggt agccacgcca gccacacctg cagcagataa gctagcaaat 540
acgacgcctg caacggacaa ggcagtagcc acaacgccag cgacgccggt agcaaataaa 600
gcagcagaca cgagtagtat tcatgatcaa ccattagata caaatgtgcc aactgataaa 660
tcagcaaacc tcgtctcgac aacacaaaaa agtacggata atcaacaagt taagtctaca 720
gaaacatctc atcttcaaga aatcaacggt aaaacctatt ttcttgacga caatggtcaa 780
gttaaaaaga acttcaccgc tattattgac ggtaaagttc tatactttga taaaacatcc 840
ggcgaattga ccgcaaatgc accgcaagtt actaagggat tagtaaatat tgataatgca 900
cataacgcgg ctcatgatct cacagctgat aacttcacaa atgtcgatgg ttacttaaca 960
gctaacagtt ggtatcgtcc taaggacatc ttaaaaaacg gaacgacctg gacaccaaca 1020
acagcagaag attttcgacc attgctcatg tcttggtggc cggataagaa tacgcaggta 1080
gcttatctac aatatatgca atcagttggt atgctacctg acgatgttaa agtatcaaat 1140
gatgataata tgagcacatt gactgatgct gctatgactg ttcaaaagaa tatcgaatcg 1200
cgaattggtg tatctggaaa aactgattgg ctcaagcaag atatgaacaa actgattgat 1260
tcacaggcaa attggaatat tgatagtgaa tcaaagggta atgatcattt acagggtggg 1320
gcattgttat atgtgaatga tgacaaaaca cctaacgcga actcagatta ccgtctgtta 1380
aaccgtacac caaccaacca aaccggccaa attactgatc caagtaaaca aggtggatat 1440
gagatgttat tagctaatga tgttgataat tctaaccctg ttgtacaagc tgagcaattg 1500
aactggcttc actacatgat gaacattggt actatagctc agaacgaccc aacagctaat 1560
tttgacggtt atcgtgttga tgcggttgat aacgttgatg ccgatctctt acaaattgct 1620
ggtgattact ttaaagctgc atacggtact ggtaaaactg aggcaaacgc aaacaatcat 1680
atttcgatct tggaagattg ggataataat gattctgcgt acattaaagc ccacgggaat 1740
aaccaattga caatggattt tccagcacac ttggctttga aatacgcctt gaacatgcct 1800
cttgccgcac aaagtggcct agaaccgcta attaatacaa gtcttgttaa gcgtgggaaa 1860
gatgccacag aaaatgaagc acaaccaaac tatgccttta tccgtgccca tgatagtgaa 1920
gtgcagaccg ttattgcaca aattattaag gataaaatta acacaaaatc agacggctta 1980
actgtaacac cagatgagat taagcaagct ttcactattt acaacgccga tgaattaaaa 2040
gcagataagg aatatacagc atacaatatt cctgcttctt acgctgtatt gttgacaaac 2100
aaggatactg tgccacgtgt ttattatggt gatctatttt ctgatgatgg acagtatatg 2160
tcacagaagt caccatacta tgacgccatt acgtcacttt tgaaaagccg tatcaaatat 2220
gttgctggtg gtcaaagtat gaatatgacg tacttgcatg agtgctttga tccagcaaaa 2280
aatgagacaa agccacaagg tgtcttaaca tcagtacgtt acggtaaagg tgcgatgacg 2340
gctgacgatt tgggtaatag tgacacacgt caacaaggta ttggtttggt gattaataat 2400
aagccattct tgaatttaaa tgatgatgaa caaattgtgc tcaatatggg tgctgctcac 2460
aaaaatcaag cttaccgacc acttatgttg acaacaaaat ctggtcttca aatttacgat 2520
aaggatgccg gagcgccagt tgtttatact aacgatgctg gtcaacttat ttttaagtca 2580
gatatggtct atggtgtcag caatccacag gtatctggtt attttgctgc atgggtacca 2640
gtcggtgcga gtgatagtca agatgctaga acacaaagca gccagtcaga aactaaggat 2700
ggcgatgtct atcattcaaa tgctgcgctt gattctaatg tgatttatga aggcttctcg 2760
aatttccaag caatgcctga aaagaatgat gacttcacca acgtaaaaat tgctcaaaat 2820
gctaaattgt ttaaagattt agggattaca agctttgaat tagcaccgca atatcgttca 2880
agtacagata atagtttttt ggattcggtt atccaaaacg gctatgcctt tactgatcga 2940
tatgatgttg gctataatac gccaacaaaa tatggtacag ttgatcaact tctagatagt 3000

ctaagagcat tacacgcaca aggtattcag gctattaatg actgggtacc tgatcaaatt 3060
tataatttac ctggcgaaca aatcgtcacc gcagttcgta caaatggttc aggtaagtac 3120
gattatgatt cagtgattaa taacacgctc tatgattcac gaacagttgg gggcggcgaa 3180
taccaagaaa agtttggtgg cctgttctta gaccagttga aaaaagatta tcctagcttg 3240
tttgaaacta agcagatatc aacgaatcag ccgatgaatc cggatgttaa aattaaagaa 3300
tggtctgcaa agtactttaa tggttcaaac attcaaggtc gtggcgcttg gtatgtactt 3360
aaagactggg caacaaatca atatttcaat gtgtctagtg ataatggatt cttgcctaaa 3420
cagttactgg gtgaaaaaac aagcaccggc tttataacag aaaatggtaa gacttctttc 3480
tactcaacaa gtggttatca agctaaagat acctttattc aagatggaac aaattggtat 3540
tactttgata atgcaggcta tatgttgaca ggtaaacaaa atatccacga taaaaattat 3600
tatttcttac ctaatggtgt ggaacttcaa gatgcttacc tttttgatgg taatcaagaa 3660
ttttactata ataaagctgg ggaacaagtt atgaaccagt attatcaaga tagtcaaaat 3720
caatggcatt atttctttga aaatggtcgc atggcaattg gcctgacaga agttccgaac 3780
gctgatggca cccatgttac acaatatttt gatgctaatg gtgtccaaat taaaggcaca 3840
gctataaaag atcagaataa tcaattacgc tattttgatg aggccacagg taatatggtg 3900
gttaattcat ggggacagtt agcagataag tcttggcttt accttaatgc acaaggcgtt 3960
gctgtgactg gtaaccaaaa aattgatggt gaagagtact acttcaatgc tgatggtaag 4020
caagttaaag gcaatgcaat catcgataat aatggtgatc aacgttatta tgatggtgat 4080
aagggtgtca tggtagttaa ttcatggggt gagttgccag atggctcatg gttatatttg 4140
aatgacaaag gtattgctgt aacaggccgt caagtcatta ataatcaagt taatttcttt 4200
ggtaatgatg gtaagcaaat caaagatgcc tttaaattat tatccgatgg ttcatgggtg 4260
tatttggatg ataagggcct gataacaact ggagccaaag ttatcaatgg tctaaatatg 4320
tttttgata aagacggtca tcaaatcaaa ggtgatgcca gcacggatgc caatggtaag 4380
cgccattatt atgacaaaaa tgatggtcat cttgtcacaa attcatgggg tgagttgcca 4440
gatggttcat ggttatatct agaagaacaa ggtgatgctg ttactggtca acgtgtgatt 4500
gatggcaaga cacgctattt tgatgaagat ggcaaacaaa ttaaaaatag cctaaaaacg 4560
ctggccaatg gcgataagat ttatcttgat ggtgatgggg ttgctgcaac aggcttacaa 4620
catgtgggcg ataaaatcat gtattttgat gaagatggca aacaagttgt tggcaagttt 4680
gtatcagcaa aagatggttc atggtattac ttaaatcagg atggtgttgc cgcggttggt 4740
ccaagcagca ttaatggaca atcactttac tttgatcaag atggtaaaca agttaaatat 4800
aatgaagttc gtaatagtga tggaacaacc aactattaca caggattaac gggtgaaaag 4860
ttaacgcaag acttcggtga actaccagat ggttcatgga tttatcttga tgcgcaaggt 4920
catacagtaa ctggtgcaca aatcattaac ggtcaaaatc tttactttaa ggctgacggc 4980
cagcaagtta aaggtcatgc ttatactgac caattaggtc atatgcgttt ttatgatcct 5040
gattcaggtg atatgttgag taatcgcttt gaacaaatca cacctggtgt atgggcttac 5100
tttggtgctg atggtgtggc cataactgga caacatgaca taaatggtca gaagctattc 5160
tttgatgaga caggatatca agttaaaggt tcgcaacgta caatagatgg tacgttatac 5220
agcttcgatt ctcaaactgg taaccaaaaa cgcgtacaga caacattgtt gccacaagca 5280
ggtcactata tcacgaaaaa tggtaacgat tggcagtatg ataccaatgg tgaactagcg 5340
aagggtctgc gtcaagatag caatggtaag ttgcgttact ttgatttgac aaccggcata 5400
caagcgaaag gccaatttgt tacaattggc caagaaactt attactttag taaagatcac 5460
ggggatgcgc agttattgcc aatggtcact gaagggcatt acggtacaat aacactcaag 5520
caaggtcaag acaccaaaac agcctgggtt taccgtgatc aaaataatac tattttgaag 5580
ggattgcaaa atatcaatgg cacgttgcaa ttctttgatc catatacagg tgaacaactt 5640
aagggtggcg tagcaaagta tgacgacaag ctcttttact ttgaatcagg taaaggtaat 5700
cttgttagca ccgtagcagg tgactatcag gatggtcatt atatttccca agatggccaa 5760
acacgttacg cagataagca aaatcagctt gtaaagggac ttgttactgt taatggggca 5820
ttacaatact ttgataacgc tactggtaac caaataaaaa atcaacaagt tattgttgat 5880
ggcaagacgt actattttga cgataaaggc aatggtgaat acttattcac taatacatta 5940
gatatgtcta ctaatgcttt ttctaccaaa aatgttgcat tcaatcatga cagtagcagt 6000
ttcgaccata ctgttgatgg cttcttgacg gcagatactt ggtatcgacc aaagtcaatt 6060
ttggctaacg ggacaacttg gcgtgattcg actgataagg atatgcgacc attaatcact 6120
gtttggtggc caaataagaa tgttcaagtc aactacctca acttcatgaa agcaaatggc 6180
ttgttgacaa cagcagcaca atacacacta cattcagatc aatatgattt gaaccaagct 6240
gcacaagatg ttcaagtggc cattgaaagg cgcattgcgt cagagcatgg cacagactgg 6300
ttacagaaat tgttgtttga atcacaaaat aataacccat catttgtgaa gcaacaattc 6360
atttggaaca aggattctga atatcatggt ggtggtgatg cttggttcca aggtggttat 6420
ctgaagtatg gcaataaccc actcacacca acaactaatt ctgattatcg tcaacctggt 6480
aatgcatttg atttcttgct agccaacgac gtggataatt ctaatcctgt tgtgcaagct 6540
gaaaacttaa actggttaca ttacttaatg aactttggca ccatcactgc gggtcaagat 6600
gacgctaatt ttgatagtat tcgtattgac gctgtcgact ttattcataa tgatacaatc 6660

```
caacgtactt atgattatct tcgtgatgct tatcaagtgc aacaaagtga agccaaagca 6720
aaccagcaca tttcattggt tgaagctggc ttagacgcag gtacatcaac gattcataat 6780
gatgcgttaa ttgagtcaaa cctccgtgaa gcagcgacat tgtcgttaac aaatgaacct 6840
ggtaaaaata aaccattgac gaatatgcta caagacgttg acggcggtac gcttatcacc 6900
gaccatacgc agaatagtac agaaaatcag gcgacaccaa actattcaat tattcacgcg 6960
cacgataaag gtgtgcaaga aaaagtaggt gcagccatta ctgatgctac tggtgctgat 7020
tggacgaact ttacagatga acagttaaaa gccggattag agctattcta taaggatcag 7080
cgcgcaacaa acaaaaagta taatagttat aacataccaa gtatttatgc cctgatgttg 7140
acaaacaaag atactgttcc tcgtatgtat tatggggata tgtatcaaga tgacggacag 7200
tatatggcaa acaagagtat ctactatgat gccttagtgt cattaatgac ggctcgtaaa 7260
agctatgtca gcggtggtca aactatgagt gttgacaatc atggtttgtt gaagagtgtc 7320
cgttttggaa aagatgcgat gacagctaat gatttaggta catcagctac gcgtactgag 7380
ggtcttggtg tcattattgg taatgatcca aagttgcaac ttaatgattc ggataaagtg 7440
acactggata tgggtgcagc acataaaaat caaaagtatc gcgcagttat cttaacaaca 7500
cgtgatggtt tggcaacctt taattcagat caagcaccaa cagcttggac aaacgatcaa 7560
ggaacgttaa cattcttcaa tcaagagatt aacgggcaag acaatacaca aattcgtggt 7620
gttgctaatc cgcaagtttc tggttatcta gctgtttggg tgcctgtggg tgcatcagac 7680
aatcaagatg cccgtacagc agcaacgaca acagaaaatc atgatggtaa agtattacac 7740
tcgaatgcgg cattagattc taaccttatt tatgaaggtt tctctaactt ccaacctaag 7800
gcaacaacgc atgatgaact tacgaacgtt gtaattgcta aaaatgccga tgtcttcaat 7860
aattggggta ttacgagttt tgaaatggca ccacagtacc gttcaagtgg ggaccataca 7920
ttcttggatt caacgattga taatggttat gccttcactg atcgctatga cttaggtttc 7980
aatacaccaa caaagtatgg cactgatggt gatttgcgtg caacgattca agcgctacat 8040
catgctaata tgcaagttat ggctgacgtt gttgataacc aggtctataa cttacctggt 8100
aaagaagttg tttcagcaac acgagcaggt gtttatggta atgacgacgc cacgggcttt 8160
ggaacgcaac tctatgtgac taactccgtt ggtggtggtc aataccaaga gaaatatgct 8220
ggacaatact tagaagctct gaaagcaaag tatccagacc tctttgaggg taaggcctat 8280
gattattggt ataagaacta tgcaaatgat gggtcaaatc cttactatac attgtcacac 8340
ggtgaccgtg aatctatccc agcagatgtt gctattaagc aatggtcagc taagtatatg 8400
aacggcacga acgttttggg caatggtatg ggttatgtat tgaaggattg gcataatggt 8460
caatatttca agcttgatgg tgataaatca acattacctc aaattt                8506
```

<210> 5
<211> 8931
<212> ADN
<213> Gène dsr-E

<400> 5

```
aataatctgt ctccattgct ttcaaaataa taatagttaa ttattatcat ggaacaatca 60
atattttatt tatattcact attgaatatc ctttttttgca taaatctcta gagccgattt 120
tttgggttat acaatgaatt ggtaaaggtt aatcattttt acaaaaccat ggtggttttt 180
tattttttct aaaattaccg aactagagga agagaaaagg agcaatagtt gtatgagaga 240
catgagggta atttgtgacc gtaaaaaatt gtacaaatcg ggcaaagtac tagtaacagc 300
cggtattttt gctttgatga tgtttggcgt cacaactgct agtgttagtg caaatacgat 360
tgcagttgac acgaatcata gccgtacttc agcacagatt aataagagtg ccgttgataa 420
ggttaatgat gacaagacta ctttaggagc ggcaaaagta gtggcagtag ccacaacgcc 480
agcgacaccg gtagcagata aaacagtaag tgcacccgca gcagataagg cagtagatac 540
aacgtcatca acgacacctg caacggataa ggcagtagat acaacgccaa cgacacctgc 600
agcagataag gcagtagata caacgccaac gacacctgca gcagataagg cagtagatac 660
aacgccaacg acacctgcag caaataaagc agtagataca acgccagcga ccgctgcaac 720
agataaggcg gtagccacgc cagccacacc tgcagcagat aagctagcaa atacgacgcc 780
tgcaacggac aaggcagtag ccacaacgcc agcgacgccg gtagcaaata aagcagcaga 840
cacgagtagt attcatgatc aaccattaga tacaaatgtg ccaactgata aatcagcaaa 900
cctcgtctcg acaacacaaa aaagtacgga taatcaacaa gttaagtcta cagaaacatc 960
tcatcttcaa gaaatcaacg gtaaaaccta ttttcttgac gacaatggtc aagttaaaaa 1020
gaacttcacc gctattattg acggtaaagt tctatacttt gataaaacat ccggcgaatt 1080
gaccgcaaat gcaccgcaag ttactaaggg attagtaaat attgataatg cacataacgc 1140
ggctcatgat ctcacagctg ataacttcac aaatgtcgat ggttacttaa cagctaacag 1200
ttggtatcgt cctaaggaca tcttaaaaaa cggaacgacc tggacaccaa caacagcaga 1260
```

agattttcga ccattgctca tgtcttggtg gccggataag aatacgcagg tagcttatct 1320
acaatatatg caatcagttg gtatgctacc tgacgatgtt aaagtatcaa atgatgataa 1380
tatgagcaca ttgactgatg ctgctatgac tgttcaaaag aatatcgaat cgcgaattgg 1440
tgtatctgga aaaactgatt ggctcaagca agatatgaac aaactgattg attcacaggc 1500
aaattggaat attgatagtg aatcaaaggg taatgatcat ttacagggtg gggcattgtt 1560
atatgtgaat gatgacaaaa cacctaacgc gaactcagat taccgtctgt taaaccgtac 1620
accaaccaac caaaccggcc aaattactga tccaagtaaa caaggtggat atgagatgtt 1680
attagctaat gatgttgata attctaaccc tgttgtacaa gctgagcaat tgaactggct 1740
tcactacatg atgaacattg gtactatagc tcagaacgac ccaacagcta attttgacgg 1800
ttatcgtgtt gatgcggttg ataacgttga tgccgatctc ttacaaattg ctggtgatta 1860
ctttaaagct gcatacggta ctggtaaaac tgaggcaaac gcaaacaatc atatttcgat 1920
cttggaagat tgggataata atgattctgc gtacattaaa gcccacggga ataaccaatt 1980
gacaatggat tttccagcac acttggcttt gaaatacgcc ttgaacatgc ctcttgccgc 2040
acaaagtggc ctagaaccgc taattaatac aagtcttgtt aagcgtggga aagatgccac 2100
agaaaatgaa gcacaaccaa actatgcctt tatccgtgcc catgatagtg aagtgcagac 2160
cgttattgca caaattatta aggataaaat taacacaaaa tcagacggct taactgtaac 2220
accagatgag attaagcaag ctttcactat ttacaacgcc gatgaattaa aagcagataa 2280
ggaatataca gcatacaata ttcctgcttc ttacgctgta ttgttgacaa acaaggatac 2340
tgtgccacgt gtttattatg gtgatctatt ttctgatgat ggacagtata tgtcacagaa 2400
gtcaccatac tatgacgcca ttacgtcact tttgaaaagc cgtatcaaat atgttgctgg 2460
tggtcaaagt atgaatatga cgtacttgca tgagtgcttt gatccagcaa aaaatgagac 2520
aaagccacaa ggtgtcttaa catcagtacg ttacggtaaa ggtgcgatga cggctgacga 2580
tttgggtaat agtgacacac gtcaacaagg tattggtttg gtgattaata ataagccatt 2640
cttgaattta aatgatgatg aacaaattgt gctcaatatg ggtgctgctc acaaaaatca 2700
agcttaccga ccacttatgt tgacaacaaa atctggtctt caaatttacg ataaggatgc 2760
cggagcgcca gttgtttata ctaacgatgc tggtcaactt atttttaagt cagatatggt 2820
ctatggtgtc agcaatccac aggtatctgg ttattttgct gcatgggtac cagtcggtgc 2880
gagtgatagt caagatgcta gaacacaaag cagccagtca gaaactaagg atggcgatgt 2940
ctatcattca aatgctgcgc ttgattctaa tgtgatttat gaaggcttct cgaatttcca 3000
agcaatgcct gaaaagaatg atgacttcac caacgtaaaa attgctcaaa atgctaaatt 3060
gtttaaagat ttagggatta caagctttga attagcaccg caatatcgtt caagtacaga 3120
taatagtttt ttggattcgg ttatccaaaa cggctatgcc tttactgatc gatatgatgt 3180
tggctataat acgccaacaa aatatggtac agttgatcaa cttctagata gtctaagagc 3240
attacacgca caaggtattc aggctattaa tgactgggta cctgatcaaa tttataattt 3300
acctggcgaa caaatcgtca ccgcagttcg tacaaatggt tcaggtaagt acgattatga 3360
ttcagtgatt aataacacgc tctatgattc acgaacagtt gggggcggcg aataccaaga 3420
aaagtttggt ggcctgttct tagaccagtt gaaaaaagat tatcctagct tgtttgaaac 3480
taagcagata tcaacgaatc agccgatgaa tccggatgtt aaaattaaag aatggtctgc 3540
aaagtacttt aatggttcaa acattcaagg tcgtggcgct tggtatgtac ttaaagactg 3600
ggcaacaaat caatatttca atgtgtctag tgataatgga ttcttgccta aacagttact 3660
gggtgaaaaa acaagcaccg gctttataac agaaaatggt aagacttctt tctactcaac 3720
aagtggttat caagctaaag ataccttat tcaagatgga acaaattggt attactttga 3780
taatgcaggc tatatgttga caggtaaaca aaatatccac gataaaaatt attatttctt 3840
acctaatggt gtggaacttc aagatgctta ccttttgat ggtaatcaag aattttacta 3900
taataaagct ggggaacaag ttatgaacca gtattatcaa gatagtcaaa atcaatggca 3960
ttatttcttt gaaaatggtc gcatggcaat tggcctgaca gaagttccga acgctgatgg 4020
cacccatgtt acacaatatt ttgatgctaa tggtgtccaa attaaaggca cagctataaa 4080
agatcagaat aatcaattac gctattttga tgaggccaca ggtaatatgg tggttaattc 4140
atggggacag ttagcagata agtcttggct ttaccttaat gcacaaggcg ttgctgtgac 4200
tggtaaccaa aaaattgatg gtgaagagta ctacttcaat gctgatggta agcaagttaa 4260
aggcaatgca atcatcgata ataatggtga tcaacgttat tatgatggtg ataagggtgt 4320
catggtagtt aattcatggg gtgagttgcc agatggctca tggttatatt tgaatgacaa 4380
aggtattgct gtaacaggcc gtcaagtcat taataatcaa gttaatttct ttggtaatga 4440
tggtaagcaa atcaaagatg cctttaaatt attatccgat ggttcatggg tgtatttgga 4500
tgataagggc ctgataacaa ctggagccaa agttatcaat ggtctaaata tgttttttga 4560
taaagacggt catcaaatca aaggtgatgc cagcacggat gccaatggta agcgccatta 4620
ttatgacaaa aatgatggtc atcttgtcac aaattcatgg ggtgagttgc cagatggttc 4680
atggttatat ctagaagaac aaggtgatgc tgttactggt caacgtgtga ttgatggcaa 4740
gacacgctat tttgatgaag atggcaaaca aattaaaaat agcctaaaaa cgctggccaa 4800
tggcgataag atttatcttg atggtgatgg ggttgctgca acaggcttac aacatgtggg 4860
cgataaaatc atgtattttg atgaagatgg caaacaagtt gttggcaagt ttgtatcagc 4920

aaaagatggt tcatggtatt acttaaatca ggatggtgtt gccgcggttg gtccaagcag 4980
cattaatgga caatcacttt actttgatca agatggtaaa caagttaaat ataatgaagt 5040
tcgtaatagt gatggaacaa ccaactatta cacaggatta acgggtgaaa agttaacgca 5100
agacttcggt gaactaccag atggttcatg gatttatctt gatgcgcaag gtcatacagt 5160
aactggtgca caaatcatta acggtcaaaa tctttacttt aaggctgacg gccagcaagt 5220
taaaggtcat gcttatactg accaattagg tcatatgcgt ttttatgatc ctgattcagg 5280
tgatatgttg agtaatcgct ttgaacaaat cacacctggt gtatgggctt actttggtgc 5340
tgatggtgtg gccataactg gacaacatga cataaatggt cagaagctat tctttgatga 5400
gacaggatat caagttaaag gttcgcaacg tacaatagat ggtacgttat acagcttcga 5460
ttctcaaact ggtaaccaaa aacgcgtaca gacaacattg ttgccacaag caggtcacta 5520
tatcacgaaa aatggtaacg attggcagta tgataccaat ggtgaactag cgaagggtct 5580
gcgtcaagat agcaatggta agttgcgtta ctttgatttg acaaccggca tacaagcgaa 5640
aggccaattt gttacaattg gccaagaaac ttattacttt agtaaagatc acggggatgc 5700
gcagttattg ccaatggtca ctgaagggca ttacggtaca ataacactca agcaaggtca 5760
agacaccaaa acagcctggg tttaccgtga tcaaaataat actattttga agggattgca 5820
aaatatcaat ggcacgttgc aattctttga tccatataca ggtgaacaac ttaagggtgg 5880
cgtagcaaag tatgacgaca agctctttta ctttgaatca ggtaaaggta atcttgttag 5940
caccgtagca ggtgactatc aggatggtca ttatatttcc caagatggcc aaacacgtta 6000
cgcagataag caaaatcagc ttgtaaaggg acttgttact gttaatgggg cattacaata 6060
ctttgataac gctactggta accaaataaa aaatcaacaa gttattgttg atggcaagac 6120
gtactatttt gacgataaag gcaatggtga atacttattc actaatacat tagatatgtc 6180
tactaatgct ttttctacca aaaatgttgc attcaatcat gacagtagca gtttcgacca 6240
tactgttgat ggcttcttga cggcagatac ttggtatcga ccaaagtcaa ttttggctaa 6300
cgggacaact tggcgtgatt cgactgataa ggatatgcga ccattaatca ctgtttggtg 6360
gccaaataag aatgttcaag tcaactacct caacttcatg aaagcaaatg gcttgttgac 6420
aacagcagca caatacacac tacattcaga tcaatatgat ttgaaccaag ctgcacaaga 6480
tgttcaagtg gccattgaaa ggcgcattgc gtcagagcat ggcacagact ggttacagaa 6540
attgttgttt gaatcacaaa ataataaccc atcatttgtg aagcaacaat tcatttggaa 6600
caaggattct gaatatcatg gtggtggtga tgcttggttc caaggtggtt atctgaagta 6660
tggcaataac ccactcacac caacaactaa ttctgattat cgtcaacctg gtaatgcatt 6720
tgatttcttg ctagccaacg acgtggataa ttctaatcct gttgtgcaag ctgaaaactt 6780
aaactggtta cattacttaa tgaactttgg caccatcact gcgggtcaag atgacgctaa 6840
ttttgatagt attcgtattg acgctgtcga ctttattcat aatgatacaa tccaacgtac 6900
ttatgattat cttcgtgatg cttatcaagt gcaacaaagt gaagccaaag caaaccagca 6960
catttcattg gttgaagctg gcttagacgc aggtacatca acgattcata atgatgcgtt 7020
aattgagtca aacctccgtg aagcagcgac attgtcgtta acaaatgaac ctggtaaaaa 7080
taaaccattg acgaatatgc tacaagacgt tgacggcggt acgcttatca ccgaccatac 7140
gcagaatagt acagaaaatc aggcgacacc aaactattca attattcacg cgcacgataa 7200
aggtgtgcaa gaaaaagtag gtgcagccat tactgatgct actggtgctg attggacgaa 7260
ctttacagat gaacagttaa aagccggatt agagctattc tataaggatc agcgcgcaac 7320
aaacaaaaag tataatagtt ataacatacc aagtatttat gccctgatgt tgacaaacaa 7380
agatactgtt cctcgtatgt attatgggga tatgtatcaa gatgacggac agtatatggc 7440
aaacaagagt atctactatg atgccttagt gtcattaatg acggctcgta aagctatgt 7500
cagcggtggt caaactatga gtgttgacaa tcatggtttg ttgaagagtg tccgttttgg 7560
aaaagatgcg atgacagcta atgatttagg tacatcagct acgcgtactg agggtcttgg 7620
tgtcattatt ggtaatgatc caaagttgca acttaatgat tcggataaag tgacactgga 7680
tatgggtgca gcacataaaa atcaaaagta tcgcgcagtt atcttaacaa cacgtgatgg 7740
tttggcaacc tttaattcag atcaagcacc aacagcttgg acaaacgatc aaggaacgtt 7800
aacattctca aatcaagaga ttaacgggca agacaataca caaattcgtg gtgttgctaa 7860
tccgcaagtt tctggttatc tagctgtttg ggtgcctgtg ggtgcatcag acaatcaaga 7920
tgcccgtaca gcagcaacga caacagaaaa tcatgatggt aaagtattac actcgaatgc 7980
ggcattagat tctaacctta tttatgaagg tttctctaac ttccaaccta aggcaacaac 8040
gcatgatgaa cttacgaacg ttgtaattgc taaaaatgcc gatgtcttca ataattgggg 8100
tattacgagt tttgaaatgg caccacagta ccgttcaagt ggggaccata cattcttgga 8160
ttcaacgatt gataatggtt atgccttcac tgatcgctat gacttaggtt tcaatacacc 8220
aacaaagtat ggcactgatg gtgatttgcg tgcaacgatt caagcgctac atcatgctaa 8280
tatgcaagtt atggctgacg ttgttgataa ccaggtctat aacttacctg gtaaagaagt 8340
tgtttcagca acacgagcag gtgtttatgg taatgacgac gccacgggct ttggaacgca 8400
actctatgtg actaactccg ttggtggtgg tcaataccaa gagaaatatg ctggacaata 8460
cttagaagct ctgaaagcaa agtatccaga cctctttgag ggtaaggcct atgattattg 8520
gtataagaac tatgcaaatg atgggtcaaa tccttactat acattgtcac acggtgaccg 8580

```
tgaatctatc ccagcagatg ttgctattaa gcaatggtca gctaagtata tgaacggcac 8640
gaacgttttg ggcaatggta tgggttatgt attgaaggat tggcataatg gtcaatattt 8700
caagcttgat ggtgataaat caacattacc tcaaatttaa tttattttga tagggaacga 8760
ttatcttatc aaattgtagt gacaaaagtc gcagatattg aatccaatat ctgcgacttt 8820
tcgtctgtaa agctatgcta taataacgtt atgacaaaag aaaattattt taaagttggc 8880
acaattgtca acacccacgg tattcgtggc gaagtgaaga ttatggatat c          8931
```

<210> 6
<211> 17
<212> PRT
<213> Peptides

<400> 6

```
Ala Asn Trp Asn Ile Asp Ser Glu Ser Lys Gly Asn Asp His Leu Gln
1               5                   10                  15

Gly
```

<210> 7
<211> 24
<212> PRT
<213> Peptides

<400> 7

```
Gly Gly Tyr Glu Met Leu Leu Ala Asn Asp Val Asp Asn Ser Asn Pro
1               5                   10                  15

Val Val Gln Ala Glu Gln Leu Asn
            20
```

<210> 8
<211> 21
<212> PRT
<213> Peptides

<400> 8

```
Ala Asn Phe Asp Gly Tyr Arg Val Asp Ala Val Asp Asn Val Asp Ala
1               5                   10                  15

Asp Leu Leu Gln Ile
            20
```

<210> 9
<211> 12
<212> PRT
<213> Peptides

<400> 9

```
His Ile Ser Ile Leu Glu Asp Trp Asp Asn Asn Asp
1               5                   10
```

<210> 10
<211> 15
<212> PRT
<213> Peptides

<400> 10

Tyr Ala Phe Ile Arg Ala His Asp Ser Glu Val Gln Thr Val Ile
1 5 10 15

<210> 11
<211> 8
<212> PRT
<213> Peptides

<400> 11

Asp Trp Val Pro Asp Gln Ile Tyr
1 5

<210> 12
<211> 19
<212> PRT
<213> Peptides

<400> 12

Phe Ile Trp Asn Lys Asp Ser Glu Tyr His Gly Gly Gly Asp Ala Trp
1 5 10 15

Phe Gln Gly

<210> 13
<211> 24
<212> PRT
<213> Peptides

<400> 13

Asn Ala Phe Asp Phe Leu Leu Ala Asn Asp Val Asp Asn Ser Asn Pro
1 5 10 15

Val Val Gln Ala Glu Asn Leu Asn
20

<210> 14
<211> 13
<212> PRT
<213> Peptides

<400> 14

Ala Asn Phe Asp Ser Ile Arg Ile Asp Ala Val Asp Phe
1 5 10

<210> 15
<211> 8
<212> PRT
<213> Peptides

<400> 15

```
His Ile Ser Leu Val Glu Ala Gly
 1               5
```

<210> 16
<211> 8
<212> PRT
<213> Peptides

<400> 16

```
Tyr Ser Ile Ile His Ala His Asp
 1               5
```

<210> 17
<211> 8
<212> PRT
<213> Peptides

<400> 17

```
Asp Val Val Asp Asn Gln Val Tyr
 1               5
```